# EUROPEAN PATENT APPLICATION

(11) **EP 2 532 656 A1**
(43) Date of publication of application: **12.12.2012**
(21) Application number: 11737210.2
(22) Date of filing: 01.02.2011
(51) Int. Cl.: C07D 401/04, A61K 31/497, A61K 31/506, A61P 3/04, A61P 3/10, A61P 43/00, C07D 401/14, C07D 451/02, C07D 471/04

(54) **GPR119 AGONIST**

(30) Priority: 01.02.2010 JP 2010020568
(71) Applicant: Nippon Chemiphar Co., Ltd., Tokyo 101-0032 (JP)
(72) Inventor: ENDO, Tsuyoshi, Misato-shi Saitama 341-0005 (JP); TAKAHASHI, Rie, Misato-shi Saitama 341-0005 (JP); TANAKA, Hiroto, Misato-shi Saitama 341-0005 (JP); KUNIGAMI, Toshihiro, Misato-shi Saitama 341-0005 (JP)
(74) Representative: Albrecht, Thomas
(86) International application number: PCT/JP2011/051972
(87) International publication number: WO 2011/093501

(57) **Abstract**

A compound represented by the following formula (I) or the formula (II) disclosed in the specification is a GPR119 agonist, and is used as an agent for treating diabetes: 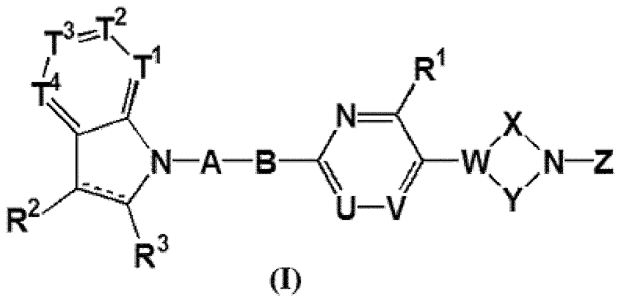 wherein one of T¹, T², T³, and T⁴ is N, and each of the other three independently is CR⁴ or, in the alternative, each of the four independently is CR⁴;
each of R² and R³ is hydrogen or a C₁₋₈ alkyl group;
the double line consisting of a solid line and a broken line means a single or double bond;
A is (CH₂)ₘ, a bond, or the like;
B is (C(R⁵)H)ₙ, a bond, or the like;
one of U and V is N, and the other is CR⁷;
W is C or CR⁸;
each of X and Y is a C₁₋₃ alkylene group, which optionally has a halogen atom, hydroxyl etc.;
Z is C(O)OR⁹, C(O)R¹⁰, or the like.

SEQUENCE LISTING

<110>  Nippon Chemiphar Co., Ltd.

<120>  GPR119 agonist

<130>  NJ-61

<150>  JP 2010-020568
<151>  2010-02-01

<160>  2

<170>  PatentIn version 3.5

<210>  1
<211>  30
<212>  DNA
<213>  Artificial Sequence

<220>
<223>  primer

<400>  1
tcctggatcc atggaatcat ctttctcatt                                        30


<210>  2
<211>  30
<212>  DNA
<213>  Artificial Sequence

<220>
<223>  primer

<400>  2
tcctgggccc ttagccatca aactctgagc                                        30

## Description

### Field of the invention

The present invention relates to a GPR119 agonist.

### Background of the invention

Diabetes is a life-style related disease and the number of patients increases all over the world. The treatments for diabetes are classified into diet, exercise and drug therapy (injectable insulin and an oral anti-diabetic drug). Some oral anti-diabetic drugs, for example, α-glucosidase inhibitors (acarbose, voglibose), insulin-sensitizing agents (pioglitazone hydrochloride), biguanides (metformin hydrochloride), sulfonylureas (glibenclamide, glimepiride), and short-acting insulin secretagogues (mitiglinide calcium hydrate) are commercially available.

Recently, an incretin mimetics (excenatide) and a DPP IV inhibitor (sitagliptin), which accelerate secretion of insulin, have been developed and are also commercially available. Further, SGLT inhibitors have been developed.

GPR119 has been reported as a G protein-coupled-receptor (GPCR) whose endogenous ligand is N-oleoylethanolamide and which stimulate insulin secretion from pancreatic β-cells (Non-patent Document 1). It has been reported that GPR119 agonist increases the plasma concentration of Glucagon like peptide-1 (GLP-1), one of incretins (Non-patent Document 2), which may indirectly relate to stimulation of insulin secretion. It has been further reported that GPR119 agonist suppresses a weight increase in rats fed a high-fat diet (Non-patent Document 1), which may relate to energy metabolism. For the reasons mentioned above, the GPR119 agonist has been expected as a drug not only for diabetes but also for life-style related diseases such as obesity and metabolic syndrome.

Compounds such as (A) are described in Patent Document 1 as the GPR119 agonist.

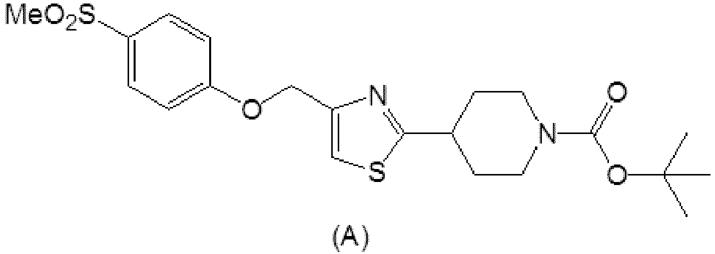

Compounds such as (B) are described in Patent Document 2 as the GPR119 agonist.

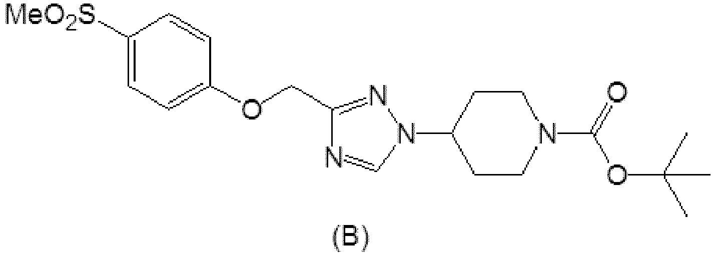

Compounds such as (C) are described in Patent Document 3 as the GPR119 agonist.

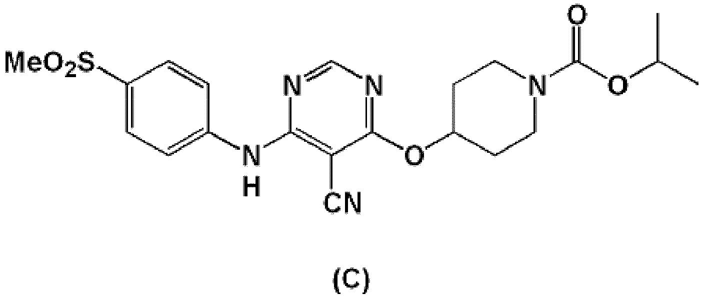

The compounds of the present invention represented by the below-described formulas (I) and (II) are different from the compounds (A) to (C) because the carbon atom of a cyclic amine such as a piperidine ring is directly combined with a pyrimidine ring or the like in the compounds of the present invention.

Compounds such as (D) are described in Patent Document 4.

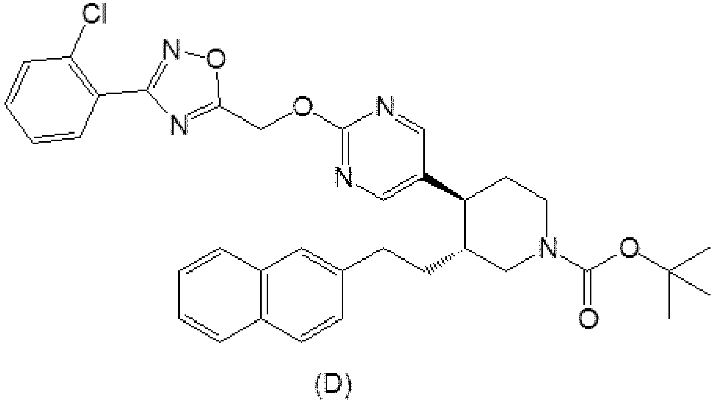

Compounds such as (E) are described in Patent Document 5.

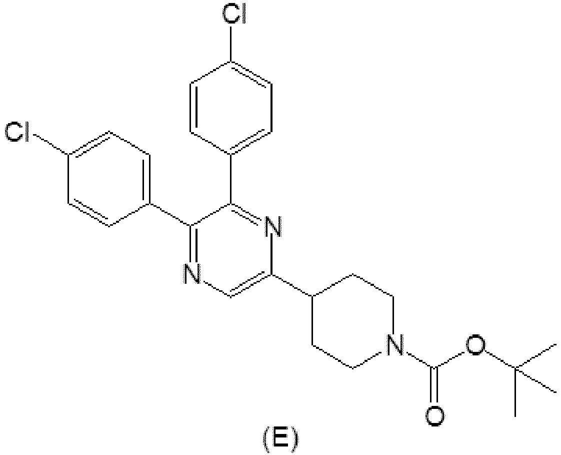

The compounds of the present invention represented by the below-described formulas (I) and (II) are clearly different from the compounds (D) and (E) in their structures. Further, there is no description in the Patent Document 4 that the compound (D) has a function of a GPR119 agonist, though the Document 4 uses the compound (D) as an intermediate in preparation of an agent for treating Alzheimer's disease. There is also no description in the Patent Document 5 that the compound (E) has a function of a GPR119 agonist, though the Document 5 describes use of the compound (E) as a cannabinoid receptor-1 (CB1) antagonist.

Patent Documents 6 and 7 have recently been disclosed as International Publications with respect to GPR119 agonists.

The compounds described in Examples of the Patent Document 6 show very low agonist activities of 33 to 73% even at a high concentration of 3 µM.

A pyrimidine ring is combined with a piperidine ring via oxygen or nitrogen atom in the compounds disclosed in the Patent Document 7, while a pyrimidine ring is directly combined with a piperidine ring in the compounds of the present invention represented by the below-described formulas (I) and (II).

The present inventors have filed Patent Document 8.

The nitrogen-containing heterocyclic ring in the center of the compound molecules disclosed in the Document 8 is a pyridine or pyridazine ring, while the corresponding ring in the compounds of the present invention represented by the below-described formulas (I) and (II) is a pyrazine or pyrimidine ring.

### Prior art documents

### Patent Documents

Patent Document 1: WO 2008/083238
Patent Document 2: WO 2009/014910
Patent Document 3: WO 2005/007647
Patent Document 4: WO 2002/076440
Patent Document 5: WO 2006/113704
Patent Document 6: WO 2010/008739
Patent Document 7: WO 2010/149685
Patent Document 8: WO 2010/013849

### Non-patent Documents

Non-patent Document 1: Overton HA et al., Cell Metab., 2006, 3, 167-75
Non-patent Document 2: Chu ZL et al., Endocrinology, 2008, 149, 2038-47

### Summary of the invention

### Problems to be solved by the invention

The object of the invention is to provide a compound represented by the formula (I) or (II), or a pharmaceutically acceptable salt thereof, and an agent for treating diabetes containing it as an active ingredient.

### Means for solving the problems

The present invention relates to a compound having the following formula (I) or a pharmaceutically acceptable salt thereof: 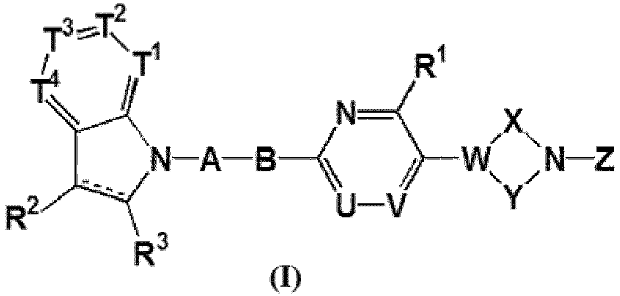
wherein one of T¹, T², T³, and T⁴ is N, and each of the other three independently is CR⁴ or, in the alternative, each of T¹, T², T³, and T⁴ independently is CR⁴, wherein R⁴ is hydrogen, a halogen atom, nitro, cyano, hydroxyl, a C₁₋₈ alkyl group, a C₁₋₈ alkoxy group, a C₁₋₈ alkyl group having one to three halogen atoms, a C₁₋₈ alkoxy group having one to three halogen atoms, phenoxy, an alkoxycarbonyl group containing a C₁₋₈ alkoxy group, carboxyl, carbamoyl, an acyl group containing a C₁₋₈ alkyl group, an alkylaminocarbonyl group containing a C₁₋₈ alkyl group, a dialkylaminocarbonyl group containing C₂₋₁₂ alkyl groups, an alkoxycarbonylmethylcarbonyl group containing a C₁₋₈ alkoxy group, an alkylsulfonylmethyl group containing a C₁₋₈ alkyl group, amino, a C₁₋₈ alkylamino group, a C₂₋₁₂ dialkylamino group, a C₁₋₈ alkylsulfonylamino group, an acylamino group containing a C₁₋₈ alkyl group, a C₁₋₈ alkylsulfinyl group, a C₁₋₈ alkylsulfonyl group, a cycloalkylsulfonyl group containing a three-membered to six-membered ring, sulfamoyl, a C₁₋₈ alkylaminosulfonyl group, a C₂₋₁₂ dialkylaminosulfonyl group, phenylsulfonyl, or a five-membered or six-membered heteroaryl group;
each of R² and R³ independently is hydrogen or a C₁₋₈ alkyl group;
the double line consisting of a solid line and a broken line means a single or double bond;
A is (CH₂)ₘ, C(O), or a bond, wherein m is an integer of 1 to 3;
B is (C(R⁵)H)ₙ, S, O, NR⁶, or a bond, wherein n is an integer of 1 to 3, and each of R⁵ and R⁶ is hydrogen, a C₁₋₈ alkyl group, or a three-membered to six-membered cycloalkyl group, provided that B is neither S, O, nor NR⁶ when A is a bond;
one of U and V is N, and the other is CR⁷, wherein R⁷ is hydrogen, a halogen atom, hydroxyl, a C₁₋₈ alkyl group, a C₁₋₈ alkoxy group, a C₁₋₈ alkyl group having one to three halogen atoms, or a C₁₋₈ alkoxy group having one to three halogen atoms;
R¹ is hydrogen, a halogen atom, hydroxyl, a C₁₋₈ alkyl group, a C₁₋₈ alkoxy group, a C₁₋₈ alkyl group having one to three halogen atoms, or a C₁₋₈ alkoxy group having one to three halogen atoms;
W is C or CR⁸, wherein R⁸ is hydrogen, a halogen atom, hydroxyl, a C₁₋₈ alkyl group, a C₁₋₈ alkoxy group, a C₁₋₈ alkyl group having one to three halogen atoms, or a C₁₋₈ alkoxy group having one to three halogen atoms;
X is a C₁₋₃ alkylene group, which optionally has a substituent or substituents selected from the group consisting of a halogen atom, hydroxyl, a C₁₋₈ alkyl group, a C₁₋₈ alkoxy group, a C₁₋₈ alkyl group having one to three halogen atoms, and a C₁₋₈ alkoxy group having one to three halogen atoms, and X may combine to W with a double bond when W is C;
Y is a C₁₋₃ alkylene group, which optionally has a substituent or substituents selected from the group consisting of a halogen atom, hydroxyl, a C₁₋₈ alkyl group, a C₁₋₈ alkoxy group, a C₁₋₈ alkyl group having one to three halogen atoms, and a C₁₋₈ alkoxy group having one to three halogen atoms;
the substituents of X and Y can be combined to form a C₁₋₃ alkylene group, which optionally has a C₁₋₈ alkyl group; and
Z is C(O)OR⁹, C(O)R¹⁰, SO₂R¹¹, C(O)NR¹²R¹³, CH₂C(O)N(R¹⁴)(R¹⁵), or a five-membered or six-membered heteroaryl group comprising carbon and nitrogen atoms and optionally comprising oxygen or sulfur atom, one of said carbon atoms combining to the nitrogen atom of the neighboring cyclic amine, and said heteroaryl group optionally having a substituent or substituents selected from the group consisting of a halogen atom, a C₁₋₈ alkyl group, a C₁₋₈ alkoxy group, a C₁₋₈ alkyl group having one to three halogen atoms, and a C₁₋₈ alkoxy group having one to three halogen atoms, wherein each of R⁹_{,} R¹⁰, R¹¹, R¹², R¹³, R¹⁴, and R¹⁵ independently is a C₁₋₈ alkyl group, a C₂₋₈ alkenyl group, a three-membered to six-membered cycloalkyl group, phenyl, or a C₁₋₈ alkyl group having phenyl.

The invention also relates to a compound having the following formula (II) or a pharmaceutically acceptable salt thereof: 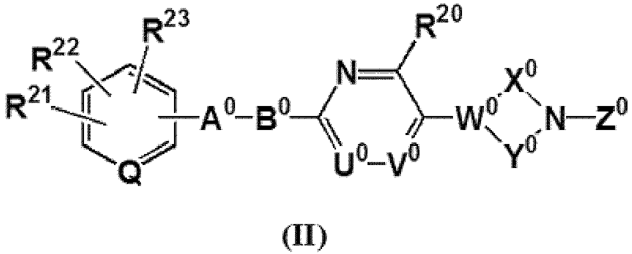
wherein each of R²¹ , R²² , and R²³ independently is hydrogen, a halogen atom, nitro, cyano, hydroxyl, a C₁₋₈ alkyl group, a C₁₋₈ alkoxy group, a C₁₋₈ alkyl group having one to three halogen atoms, a C₁₋₈ alkoxy group having one to three halogen atoms, phenoxy, an alkoxycarbonyl group containing a C₁₋₈ alkoxy group, carboxyl, carbamoyl, an acyl group containing a C₁₋₈ alkyl group, an alkylaminocarbonyl group containing a C₁₋₈ alkyl group, a dialkylaminocarbonyl group containing C₂₋₁₂ alkyl groups, an alkoxycarbonylmethylcarbonyl group containing a C₁₋₈ alkoxy group, an alkylsulfonylmethyl group containing a C₁₋₈ alkyl group, amino, a C₁₋₈ alkylamino group, a C₂₋₁₂ dialkylamino group, a C₁₋₈ alkylsulfonylamino group, an acylamino group containing a C₁₋₈ alkyl group, a C₁₋₈ alkylsulfinyl group, a C₁₋₈ alkylsulfonyl group, a cycloalkyl-sulfonyl group containing a three-membered to six-membered ring, sulfamoyl, a C₁₋₈ alkylaminosulfonyl group, a C₂₋₁₂ dialkylaminosulfonyl group, phenylsulfonyl, or a five-membered or six-membered heteroaryl group;
Q is N or CH;
A⁰ is (CH₂)ₚ, C(O), S, O, NR²⁴, or a bond, wherein p is an integer of 1 to 3, and R²⁴ is hydrogen, a C₁₋₈ alkyl group, or a three-membered to six-membered cycloalkyl group;
B⁰ is (C(R²⁵)H)_{q}, O, NR²⁶, or a bond, wherein q is an integer of 1 to 3, and each of R²⁵ and R²⁶ is hydrogen, a C₁₋₈ alkyl group, or a three-membered to six-membered cycloalkyl group, provided that B⁰ is neither O nor NR²⁶ when A⁰ is S, O, or NR²⁴, and that B⁰ is not a bond when A⁰ is a bond;
one of U⁰ and V⁰ is N and the other is CR²⁷, wherein R²⁷ is hydrogen, a halogen atom, hydroxyl, a C₁₋₈ alkyl group, a C₁₋₈ alkoxy group, a C₁₋₈ alkyl group having one to three halogen atoms, or a C₁₋₈ alkoxy group having one to three halogen atoms;
R²⁰ is hydrogen, a halogen atom, hydroxyl, a C₁₋₈ alkyl group, a C₁₋₈ alkoxy group, a C₁₋₈ alkyl group having one to three halogen atoms, or a C₁₋₈ alkoxy group having one to three halogen atoms;
W⁰ is C or CR²⁸, wherein R²⁸ is hydrogen, a halogen atom, hydroxyl, a C₁₋₈ alkyl group, a C₁₋₈ alkoxy group, a C₁₋₈ alkyl group having one to three halogen atoms, or a C₁₋₈ alkoxy group having one to three halogen atoms;
X⁰ is a C₁₋₃ alkylene group, which optionally has a substituent or substituents selected from the group consisting of a halogen atom, hydroxyl, a C₁₋₈ alkyl group, a C₁₋₈ alkoxy group, a C₁₋₈ alkyl group having one to three halogen atoms, and a C₁₋₈ alkoxy group having one to three halogen atoms, and X⁰ may combine to W⁰ with a double bond when W⁰ is C;
Y⁰ is a C₁₋₃ alkylene group, which optionally has a substituent or substituents selected from the group consisting of a halogen atom, hydroxyl, a C₁₋₈ alkyl group, a C₁₋₈ alkoxy group, a C₁₋₈ alkyl group having one to three halogen atoms, and a C₁₋₈ alkoxy group having one to three halogen atoms;
the substituents of X⁰ and Y⁰ can be combined to form a C₁₋₃ alkylene group, which optionally has a C₁₋₈ alkyl group; and
Z⁰ is C(O)OR²⁹, C(O)R³⁰, SO₂R³¹, C(O)NR³²R³³, CH₂C(O)N(R³⁴)(R³⁵), or a five-membered or six-membered heteroaryl group comprising carbon and nitrogen atoms and optionally comprising oxygen or sulfur atom, one of said carbon atoms combining to the nitrogen atom of the neighboring cyclic amine, and said heteroaryl group optionally having a substituent or substituents selected from the group consisting of a halogen atom, a C₁₋₈ alkyl group, a C₁₋₈ alkoxy group, a C₁₋₈ alkyl group having one to three halogen atoms, and a C₁₋₈ alkoxy group having one to three halogen atoms, wherein each of R²⁹, R³⁰, R³¹, R³², R³³, R³⁴, and R³⁵ independently is a C₁₋₈ alkyl group, a C₂₋₈ alkenyl group, a three-membered to six-membered cycloalkyl group, phenyl, or a C₁₋₈ alkyl group having phenyl.

The invention further relates to an agent for treating diabetes containing the compound of the formula (I) or (II) described above, or a pharmaceutically acceptable salt thereof as an active ingredient.

The invention further relates to a GPR119 agonist containing the compound of the formula (I) or (II) described above, or a pharmaceutically acceptable salt thereof as an active ingredient.

### Embodiments for conducting the invention

The present invention is described below in detail.

Preferred embodiments of the compound of the formula (I) are described below.
(1) A compound of the above-mentioned formula (I) or a pharmaceutically acceptable salt thereof, wherein each of T¹, T², T³, and T⁴ independently is CR⁴.
(2) A compound of the above-mentioned formula (I) or a pharmaceutically acceptable salt thereof, wherein T¹ is N, and each of T², T³, and T⁴ independently is CR⁴.
(3) A compound of the above-mentioned formula (I) or described in (1) or (2), or a pharmaceutically acceptable salt thereof, wherein R⁴ is hydrogen, a halogen atom, a C₁₋₈ alkyl group, cyano, an alkoxycarbonyl group containing a C₁₋₈ alkoxy group, a C₁₋₈ alkylsulfonyl group, sulfamoyl, phenylsulfonyl, or a five-membered or six-membered heteroaryl group.
(4) A compound of the above-mentioned formula (I) or described in (1) or (2), or a pharmaceutically acceptable salt thereof, wherein one of CR⁴ represented by T¹, T², T³, and T⁴ is C-(C₁₋₈ alkylsulfonyl).
(5) A compound of the above-mentioned formula (I) or described in (1) or (2), or a pharmaceutically acceptable salt thereof, wherein one of CR⁴ represented by T¹, T², T³, and T⁴ is C-(C₁₋₈ alkylsulfonyl), and each of the others of CR⁴ is selected from CH, C-(C₁₋₈ alkyl), or C-(halogeno).
(6) A compound of the above-mentioned formula (I) or described in (1) or (2), or a pharmaceutically acceptable salt thereof, wherein one of CR⁴ represented by T¹, T², T³, and T⁴ is C-(1-tetrazolyl) or C-(1,2,4-triazol-1-yl).
(7) A compound of the above-mentioned formula (I) or described in (1) or (2), or a pharmaceutically acceptable salt thereof, wherein one of CR⁴ represented by T¹, T², T³, and T⁴ is C-(1-tetrazolyl) or C-(1,2,4-triazol-1-yl), and each of the others of CR⁴ is selected from CH, C-(C₁₋₈ alkyl), or C-(halogeno).
(8) A compound of the above-mentioned formula (I) or described in one of (1) to (7), or a pharmaceutically acceptable salt thereof, wherein each of R² and R³ is hydrogen.
(9) A compound of the above-mentioned formula (I) or described in one of (1) to (8), or a pharmaceutically acceptable salt thereof, wherein A is CH₂, and B is a bond.
(10) A compound of the above-mentioned formula (I) or described in one of (1) to (9), or a pharmaceutically acceptable salt thereof, wherein U is CH, and V is N.
(11) A compound of the above-mentioned formula (I) or described in one of (1) to (9), or a pharmaceutically acceptable salt thereof, wherein U is N, and V is CH.
(12) A compound of the above-mentioned formula (I) or described in one of (1) to (11), or a pharmaceutically acceptable salt thereof, wherein each of X and Y is ethylene.
(13) A compound of the above-mentioned formula (I) or described in one of (1) to (11), or a pharmaceutically acceptable salt thereof, wherein W is C, and X combines to W with a double bond.
(14) A compound of the above-mentioned formula (I) or described in one of (1) to (13), or a pharmaceutically acceptable salt thereof, wherein Z is C(O)OR⁹.
(15) A compound of the above-mentioned formula (I) or described in (14), or a pharmaceutically acceptable salt thereof, wherein R⁹ is a C₁₋₈ alkyl group.
(16) A compound of the above-mentioned formula (I) or described in one of (1) to (13), or a pharmaceutically acceptable salt thereof, wherein Z is 3-C₁₋₈ alkyl-1,2,4-oxadiazol-5-yl or 5-C₁₋₈ alkyl-1,2,4-oxadiazol-3-yl.
(17) A compound of the above-mentioned formula (I) or described in one of (1) to (13), or a pharmaceutically acceptable salt thereof, wherein Z is 5-C₁₋₈ alkylpyrimidin-2-yl.
(18) A compound of the above-mentioned formula (I) or described in one of (1) to (17), or a pharmaceutically acceptable salt thereof, wherein each of R¹ and R⁷ is hydrogen.

Preferred embodiments of the compound of the formula (II) are described below.
(19) A compound of the above-mentioned formula (II) or a pharmaceutically acceptable salt thereof, wherein Q is CH.
(20) A compound of the above-mentioned formula (II) or described in (19), or a pharmaceutically acceptable salt thereof, wherein each of R²¹, R²², and R²³ independently is hydrogen, a halogen atom, a C₁₋₈ alkyl group, cyano, an alkoxycarbonyl group containing a C₁₋₈ alkoxy group, a C₁₋₈ alkylsulfonyl group, sulfamoyl, phenylsulfonyl, or a five-membered or six-membered heteroaryl group.
(21) A compound of the above-mentioned formula (II) or described in (19), or a pharmaceutically acceptable salt thereof, wherein one of R²¹, R²², and R²³ is a C₁₋₈ alkylsulfonyl group.
(22) A compound of the above-mentioned formula (II) or described in (19), or a pharmaceutically acceptable salt thereof, wherein one of R²¹, R²², and R²³ is a C₁₋₈ alkylsulfonyl group, and each of the others is selected from hydrogen, a C₁₋₈ alkyl group, or a halogen atom.
(23) A compound of the above-mentioned formula (II) or described in (19), or a pharmaceutically acceptable salt thereof, wherein one of R²¹, R²², and R²³ is 1-tetrazolyl or 1,2,4-triazol-1-yl.
(24) A compound of the above-mentioned formula (II) or described in (19), or a pharmaceutically acceptable salt thereof, wherein one of R²¹, R²², and R²³ is 1-tetrazolyl or 1,2,4-triazol-1-yl, and each of the others is selected from hydrogen, a C₁₋₈ alkyl group, or a halogen atom.
(25) A compound of the above-mentioned formula (II) or described in one of (19) to (24), or a pharmaceutically acceptable salt thereof, wherein A⁰ is O, and B⁰ is CH₂.
(26) A compound of the above-mentioned formula (II) or described in one of (19) to (25), or a pharmaceutically acceptable salt thereof, wherein U⁰ is CH, and V⁰ is N.
(27) A compound of the above-mentioned formula (II) or described in one of (19) to (25), or a pharmaceutically acceptable salt thereof, wherein U⁰ is N, and V⁰ is CH.
(28) A compound of the above-mentioned formula (II) or described in one of (19) to (27), or a pharmaceutically acceptable salt thereof, wherein each of X⁰ and Y⁰ is ethylene.
(29) A compound of the above-mentioned formula (II) or described in one of (19) to (27), or a pharmaceutically acceptable salt thereof, wherein W⁰ is C, and X⁰ combines to W⁰ with a double bond.
(30) A compound of the above-mentioned formula (II) or described in one of (19) to (29), or a pharmaceutically acceptable salt thereof, wherein Z⁰ is C(O)OR²⁹.
(31) A compound of the above-mentioned formula (II) or described in (30), or a pharmaceutically acceptable salt thereof, wherein R²⁹ is a C₁₋₈ alkyl group.
(32) A compound of the above-mentioned formula (II) or described in one of (19) to (29), or a pharmaceutically acceptable salt thereof, wherein Z⁰ is 3-C₁₋₈ alkyl-1,2,4-oxadiazol-5-yl or 5-C₁₋₈ alkyl-1,2,4-oxadiazol-3-yl.
(33) A compound of the above-mentioned formula (II) or described in one of (19) to (29), or a pharmaceutically acceptable salt thereof, wherein Z⁰ is 5-C₁₋₈ alkylpyrimidin-2-yl.
(34) A compound of the above-mentioned formula (II) or described in one of (19) to (33), or a pharmaceutically acceptable salt thereof, wherein each of R²⁰ and R²⁷ is hydrogen.

In the compound of the above-mentioned formula (I) or (II), examples of the halogen atoms include a fluorine atom, a chlorine atom, and a bromine atom.

Examples of the C₁₋₈ alkyl groups include methyl, ethyl, propyl, isopropyl, butyl, t-butyl, pentyl, neopentyl, and hexyl.

Examples of the three-membered to six-membered cycloalkyl groups include cyclopropyl, cyclopentyl, and cyclohexyl.

Examples of the C₁₋₈ alkoxy groups include methoxy, ethoxy, and propoxy.

Examples of the C₁₋₈ alkyl groups having one to three halogen atoms include chloromethyl, fluoromethyl, 2-fluoroethyl, and trifluoromethyl. Examples of the C₁₋₈ alkoxy groups having one to three halogen atoms include fluoromethoxy and trifluoromethoxy.

Examples of the alkoxycarbonyl groups containing a C₁₋₈ alkoxy group include methoxycarbonyl and ethoxycarbonyl. Examples of the acyl groups containing a C₁₋₈ alkyl group include acetyl. Examples of the alkylaminocarbonyl groups containing a C₁₋₈ alkyl group include methylaminocarbonyl and ethylaminocarbonyl. Examples of the dialkylaminocarbonyl groups containing C₂₋₁₂ alkyl groups include dimethylaminocarbonyl and diethylaminocarbonyl. Examples of the alkoxycarbonylmethylcarbonyl groups containing a C₁₋₈ alkoxy group include methoxycarbonylmethyl-carbonyl and ethoxycarbonylmethylcarbonyl.

Examples of the alkylsulfonylmethyl groups containing a C₁₋₈ alkyl group include methanesulfonylmethyl and ethanesulfonylmethyl. Examples of the C₁₋₈ alkylamino groups include methylamino and ethylamino. Examples of the C₂₋₁₂ dialkylamino groups include dimethylamino and di-ethylamino. Examples of the C₁₋₈ alkylsulfonylamino groups include methanesulfonylamino and ethanesulfonylamino. Examples of the acylamino groups containing a C₁₋₈ alkyl group include acetylamino.

Examples of the C₁₋₈ alkylsulfinyl groups include methylsulfinyl and ethylsulfinyl. Examples of the C₁₋₈ alkylsulfonyl groups include methanesulfonyl and ethanesulfonyl. Examples of the C₁₋₈ alkylaminosulfonyl groups include methylaminosulfonyl and ethylaminosulfonyl. Examples of the C₂₋₁₂ dialkylaminosulfonyl groups include di-methylaminosulfonyl and diethylaminosulfonyl.

Examples of the C₁₋₈ alkyl groups having phenyl include benzyl.

Examples of the C₂₋₈ alkenyl groups include vinyl and propenyl.

In the formula (I), the bicyclic heterocyclic ring comprising T¹ to T⁴ can be pyrrolopyridines such as pyrrolo[2,3-b]pyridine, pyrrolo[3,2-b]pyridine, and pyrrolo[2,3-c]pyridine, in the case that one of T¹, T², T³, and T⁴ is N, and each of the other three independently is CR⁴.

Examples of the five-membered or six-membered heteroaryl groups of R²¹, R²², or R²³ in the formula (II) include 1,2,4-triazolyl and tetrazolyl.

Examples of the five-membered or six-membered heteroaryl groups (comprising carbon and nitrogen atoms and optionally comprising oxygen or sulfur atom, one of said carbon atoms combining to the nitrogen atom of the neighboring cyclic amine) of Z in the formula (I) and Z⁰ in the formula (II) include pyrimidinyl and oxadiazolyl.

Examples of the pharmaceutically acceptable salts of the compound of the formula (I) or (II) include a salt with an inorganic acid such as a hydrochloride or a sulfate and a salt with an organic acid such as a fumarate or a methanesulfonate.

In the present invention, the compound of the formula (I) or (II) includes a racemic mixture and optically active isomers.

In the present invention, the compound of the formula (I) or (II) includes a hydrate and a solvate.

Processes for preparation of the compound of the formula (I) are described below.

A process for preparation of a compound in which A is CH₂, B is a bond, W is CH or C, X is CH₂CH₂ or CHCH₂, Y is CH₂CH₂ is below exemplified. The other analogous compounds can also be prepared according to similar processes.

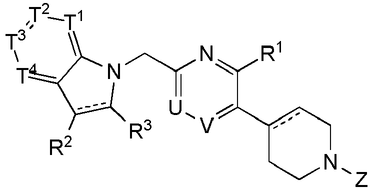

### <Method A>

### (First process)

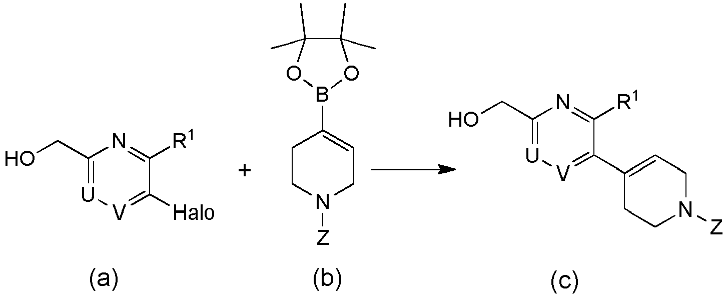

### (Second process)

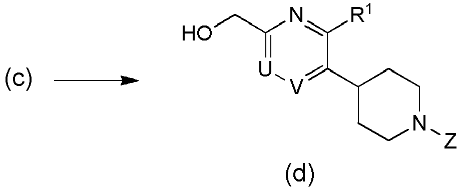

### (Third process)

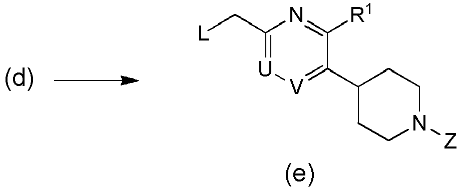

### (Fourth process)

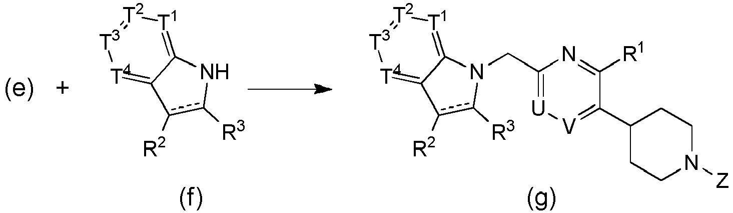

In the formulas, Halo is halogen such as chlorine, bromine, and iodine, L is halogen such as chlorine, bromine, and iodine, or a leaving group such as methanesulfonyloxy and p-toluenesulfonyloxy, and each of R¹, R², R³, T¹, T², T³, T⁴, U, V, and Z is described above.

### 1) Starting materials

The starting material (a) can be synthesized according to a known method (cf., Dong Han. Kim et. al., J. Org. Chem., 1970, 35, 455; and WO 2008/130320) or an analogous method thereof. The starting material (b) can be synthesized according to a known method (cf., G. Shyamali et. al., Can. J. Chem., 2006, 84, 555; and WO 2007/081995) or an analogous method thereof.

### 2) First process

The reaction of the starting material (a) with the starting material (b) can be conducted in an inert solvent such as toluene, tetrahydrofuran, dioxane, and N,N-dimethylformamide, in the presence of a base such as potassium carbonate, cesium carbonate, and sodium carbonate, using a catalyst such as tetrakis(triphenylphosphine)palladium and [1,1'-bis(diphenylphosphino)-ferrocene]palladium(II) dichloride dichloromethane complex to give the compound of the formula (c). The reaction temperature ranges from 20°C to 110°C.

### 3) Second process

The compound of the formula (c) can be converted into the compound of the formula (d) in an inert solvent such as methanol and ethanol, in the presence of a catalyst such as palladium-carbon according to a catalytic hydrogenation method.

### 4) Third process

The compound of the formula (d) can be converted into the compound of the formula (e) by a reaction of the compound (d) with a reagent such as methanesulfonyl chloride, p-toluenesulfonyl chloride, and thionyl chloride, in an inert solvent such as toluene and dichloromethane, optionally in the presence of a base such as pyridine and triethylamine.

### 5) Fourth process

The compound of the formula (e) can be converted into the compound of the formula (g) by a reaction of the compound (e) with the compound of the formula (f) in an inert solvent such as toluene, N,N-dimethylformamide, and acetone, in the presence of a base such as potassium hydroxide, sodium hydroxide, and potassium carbonate, optionally in the presence of an additive such as a crown ether. The reaction temperature ranges from the room temperature to 130°C.

The compound of the formula (g) can also be synthesized according to the following method B.

### <Method B>

### (First process)

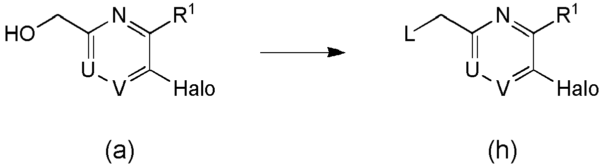

### (Second process)

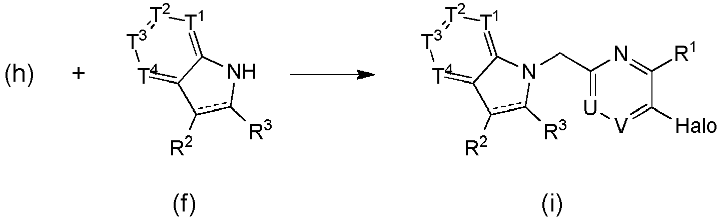

### (Third process)

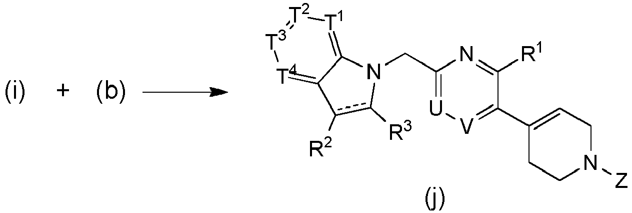

### (Fourth process)

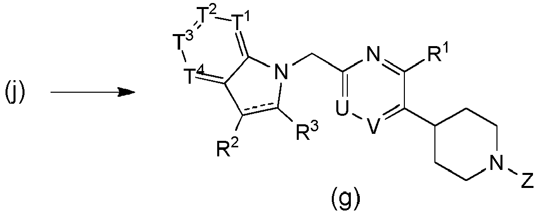

In the formulas, Halo is halogen such as chlorine, bromine, and iodine, L is halogen such as chlorine, bromine, and iodine, or a leaving group such as methanesulfonyloxy and p-toluenesulfonyloxy, and each of R¹, R², R³, T¹, T², T³, T⁴, U, V, and Z is described above.

### 1) First process

The starting material (a) can be converted into the compound of the formula (h) in the same manner as in the process of the above-mentioned method A.

### 2) Second process

The compound of the formula (h) can be converted into the compound of the formula (i) in the same manner as in the process of the above-mentioned method A.

### 3) Third process

The compound of the formula (i) can be converted into the compound of the formula (j) in the same manner as in the process of the above-mentioned method A.

### 4) Fourth process

The compound of the formula (j) can be converted into the compound of the formula (g) in the same manner as in the process of the above-mentioned method A.

Processes for preparation of the compound of the formula (II) are described below.

A process for preparation of a compound in which A⁰ is O, B⁰ is CH₂, W⁰ is CH or C, X⁰ is CH₂CH₂ or CHCH₂, Y⁰ is CH₂CH₂ is below exemplified. The other analogous compounds can also be prepared according to similar processes.

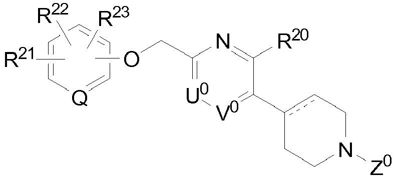

### <Method C>

### (First process)

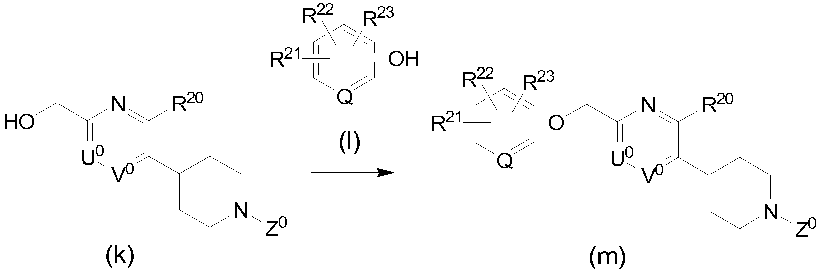

In the formulas, each of R²⁰, R²¹, R²², R²³, Q, U⁰, V⁰, and Z⁰ is described above.

### 1) Starting material

The starting material (k) can be prepared in the same manner as in the process of the above-mentioned method A.

### 2) First process

The compound of the formula (k) can be converted into the compound of the formula (m) by a reaction of the compound (k) with a phenol or heteroaryl alcohol of the formula (1) in an inert solvent such as tetrahydrofuran, dioxane, and toluene, in the presence of an azodicarboxylic ester such as diethyl azodicarboxylate, diisopropyl azodicarboxylate, and in the presence of a reagent such as triphenylphosphine. The reaction temperature ranges from 0°C to 80°C.

The compound of the formula (k) can also be converted into the compound of the formula (m) according to the following method D.

### <Method D>

### (First process)

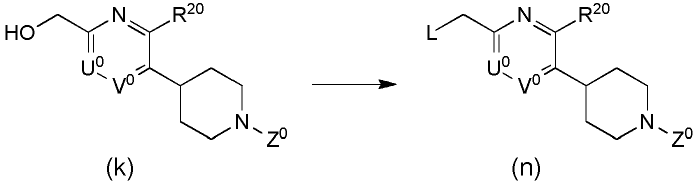

### (Second process)

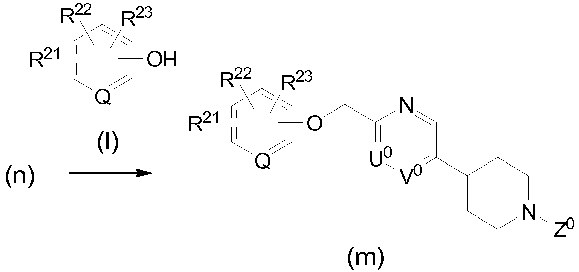

In the formulas, L is a halogen atom such as chlorine atom, bromine atom, iodine atom, or a leaving group such as methanesulfonyloxy and p-toluenesulfonyloxy, and each of R²⁰, R²¹, R²², R²³, Q, U⁰, V⁰, and Z⁰ is described above.

### 1) First process

The compound of the formula (k) can be converted into the compound of the formula (n) in the same manner as in the process of the above-mentioned method A.

### 2) Second process

The compound of the formula (n) can be converted into the compound of the formula (m) by a reaction of the compound (n) with a phenol or heteroaryl alcohol of the formula (1) in an inert solvent such as N,N-dimethylformamide and acetone, in the presence of a base such as sodium hydride and potassium carbonate. The reaction temperature ranges from 0°C to 80°C.

The compound of the formula (m) can also be prepared according to the following method E.

### <Method E>

### (First process)

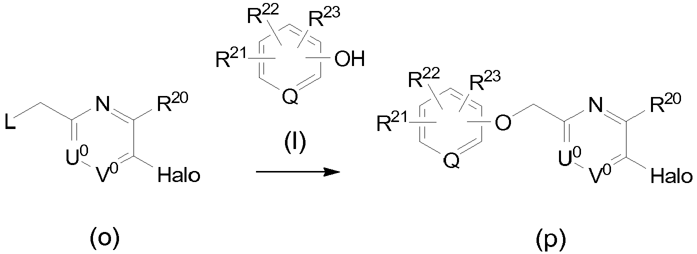

### (Second process)

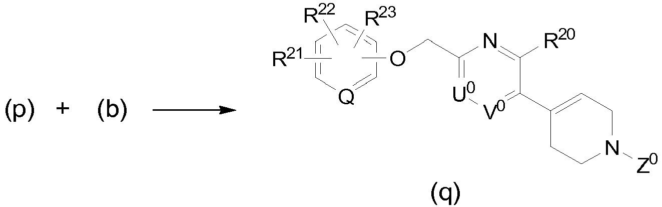

### (Third process)

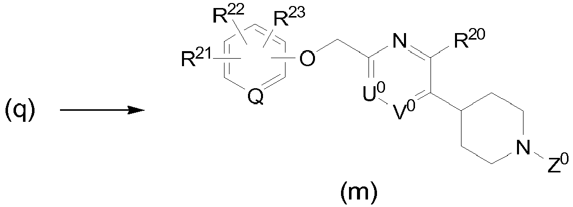

In the formulas, Halo is halogen such as chlorine, bromine, and iodine, L is halogen such as chlorine, bromine, and iodine, or a leaving group such as methanesulfonyloxy and p-toluenesulfonyloxy, and each of R²⁰, R²¹, R²², R²³, Q, U⁰, V⁰, and Z⁰ is described above.

### 1) Starting material

The starting material (o) can be synthesized in the same manner as in the process of the above-mentioned method A.

### 2) First process

The starting material (o) can be converted into the compound of the formula (p) in the same manner as in the process of the above-mentioned method D.

### 3) Second process

The compound of the formula (p) can be converted into the compound of the formula (q) in the same manner as in the process of the above-mentioned method A.

### 4) Third process

The compound of the formula (q) can be converted into the compound of the formula (m) in the same manner as in the process of the above-mentioned method A.

The compound represented by the formula (I) or (II) can also be prepared, for example by referring to the above-described methods, the below-described examples, and the Patent Documents 1 to 5.

Examples of the representative compounds of the present invention are shown below.

### Representative compound (1)

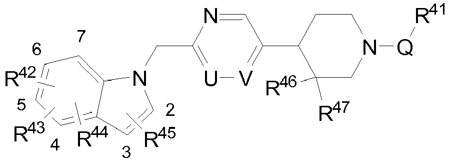

In the formula, R⁴², R⁴³, R⁴⁴ R⁴⁵, R⁴⁶ R⁴⁷, U, V, Q, and R⁴¹ are set forth in Tables 1 to 3.

**TABLE 1**

| R⁴² | R⁴³ | R⁴⁴ | R⁴⁵ | R⁴⁶ | R⁴⁷ | U | V | Q | R⁴¹ |
|---|---|---|---|---|---|---|---|---|---|
| 5-(Tetrazol-1-yl) | 7-F | - | - | H | H | CH | N | C(O)O | Isopropyl |
| 5-SO₂CH₃ | 6-F | 7-F | - | CH₃ | H | N | CH | C(O)O | t-Butyl |
| 5-SO₂CH₃ | 7-F | - | - | CH₃ | CH₃ | N | CH | C(O)O | t-Butyl |
| 5-(1,2,4-Triazol-1-yl) | - | - | - | H | H | N | CH | C(O)O | Isopropyl |
| 5-SO₂NH₂ | - | - | 3-CH₃ | CH₃ | H | CH | N | C(O) | Isobutyl |
| 5-SO₂C₂H₅ | 6-CH₃ | - | - | H | H | CH | N | C(O)O | n-Propyl |
| 4-F | 5-SO₂CH₃ | 6-F | - | CH₃ | CH₃ | CH | N | C(O)O | Ethyl |
| 5-CN | 6-F | - | 2-CH₃ | H | H | CH | N | SO)₂ | Isopropyl |
| 5-(Tetrazol-1-yl) | 7-F | - | - | CH₃ | CH₃ | CH | N | C(O)O | t-Butyl |
| 5-(1,2,4-Triazol-1-yl) | 7-F | - | - | H | H | CH | N | C(O)O | Isopropyl |

**TABLE 2**

| R⁴² | R⁴³ | R⁴⁴ | R⁴⁵ | R⁴⁶ | R⁴⁷ | U | V | Q | R⁴¹ |
|---|---|---|---|---|---|---|---|---|---|
| 4-F | 5-SO₂CH₃ | - | - | H | H | CH | N | C(O)O | Cyclopropyl |
| 5- (Tetrazol-1-yl) | 7-F | - | - | CH₃ | CH₃ | N | CH | C(O)O | t-Butyl |
| 5-SO₂CH₃ | - | - | - | CH₃ | H | CH | N | C(O)O | t-Butyl |
| 5-CO₂CH₃ | 7-CF₃ | - | - | H | H | N | CH | C(O)O | Benzyl |
| 5-SO₂CH₃ | 7-Cl | - | - | CH₃ | H | CH | N | C(O)O | t-Butyl |
| 5-SO₂CH₃ | 6-Cl | - | - | H | H | N | CH | C(O)O | n-Butyl |
| 5-SO₂NH₂ | - | - | - | H | H | CH | N | C(O)O | t-Butyl |

**TABLE 3**

| R⁴² | R⁴³ | R⁴⁴ | R⁴⁵ | R⁴⁶ | R⁴⁶ | U | V | Q | R⁴¹ |
|---|---|---|---|---|---|---|---|---|---|
| 5-SO₂CH₃ | 6-F | 7-F | - | H | H | CH | N | 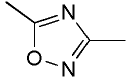 | Isopropyl |
| 5- (Tetrazol-1-yl) | 7-F | - | - | CH₃ | H | N | CH | 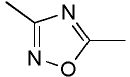 | Isopropyl |
| 5-(1,2,4-Triazol-1-yl) | - | - | - | H | H | CH | N | 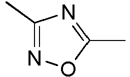 | Isopropyl |
| 5-SO₂CH₃ | - | - | - | CH₃ | CH₃ | N | CH | 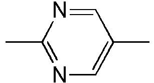 | Ethyl |
| 5-(1,2,4-Triazol-1-yl) | - | - | - | H | H | N | CH | 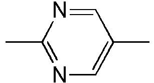 | Ethyl |
| 5- (Tetrazol-1-yl) | 7-F | - | - | H | H | N | CH | 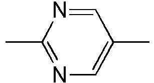 | Isopropyl |

### Representative compound (2)

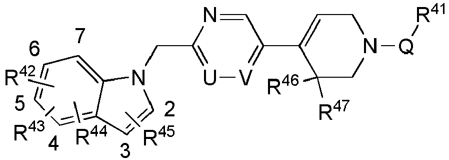

In the formula, R⁴², R⁴³, R⁴⁴, R⁴⁵, R⁴⁶, R⁴⁷, U, V, Q, and R⁴¹ are set forth in Tables 4 and 5.

**TABLE 4**

| R⁴² | R⁴³ | R⁴⁴ | R⁴⁵ | R⁴⁶ | R⁴⁷ | U | V | Q | R⁴¹ |
|---|---|---|---|---|---|---|---|---|---|
| 5-SO₂CH₃ | - | - | - | CH₃ | H | CH | N | C(O)O | Isopropyl |
| 5-(1,2,4-Triazol-1-yl) | 6-F | 7-F | - | CH₃ | H | N | CH | C(O)O | t-Butyl |
| 5-(Tetrazol-1-yl) | 7-F | - | - | CH₃ | CH₃ | CH | N | C(O)O | t-Butyl |
| 5-(1,2,4-Triazol-1-yl) | - | - | - | H | H | N | CH | C(O)O | Isopropyl |
| 5-SO₂-Cyclopropyl | - | - | 3-CH₃ | CH₃ | CH₃ | N | CH | C(O) | Isobutyl |
| 4-F | 5-SO₂CH₃ | 6-F | - | CH₃ | H | CH | N | C(O)O | Isopropyl |
| 5-(Tetrazol-1-yl) | 7-F | - | - | CH₃ | H | CH | N | C(O)O | Isopropyl |
| 5-SO₂CH₃ | 7-Cl | - | - | CH₃ | H | CH | N | C(O)O | t-Butyl |
| 5-SO₂CH₃ | 6-CH₃ | - | - | H | H | CH | N | C(O)O | n-Butyl |
| 5-(1,2,4-Triazol-1-yl) | 7-F | - | - | CH₃ | H | CH | N | C(O)O | Isopropyl |
| 5-(Tetrazol-1-yl) | 6-F | - | - | CH₃ | CH₃ | CH | N | C(O)O | t-Butyl |

**TABLE 5**

| R⁴² | R⁴³ | R⁴⁴ | R⁴⁵ | R⁴⁶ | R⁴⁷ | U | V | Q | R⁴¹ |
|---|---|---|---|---|---|---|---|---|---|
| 5- (Tetrazol-1-yl) | 7-F | - | - | H | H | CH | N | 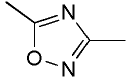 | Isopropyl |
| 5-SO₂CH₃ | 6-F | 7-F | - | CH₃ | H | N | CH | 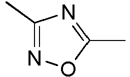 | Isopropyl |
| 5-(1,2,4-Triazol-1-yl) | - | - | - | CH₃ | H | CH | N | 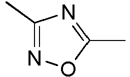 | Isopropyl |
| 5-SO₂CH₃ | - | - | - | CH₃ | CH₃ | CH | N | 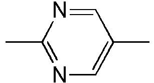 | Ethyl |
| 5-(1,2,4-Triazol-1-yl) | - | - | - | H | H | N | CH | 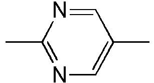 | Ethyl |
| 5- (Tetrazol-1-yl) | 7-F | - | - | CH₃ | H | CH | N | 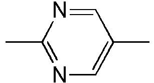 | Ethyl |

### Representative compound (3)

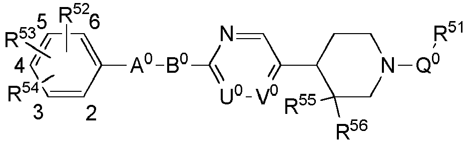

In the formula, R⁵², R⁵³, R⁵⁴, R⁵⁵, R⁵⁶, A⁰, B⁰, U⁰, V⁰, Q⁰, and R⁵¹ are set forth in Tables 6 and 7.

**TABLE 6**

| R⁵² | R⁵³ | R⁵⁵ | A⁰ | U⁰ | Q⁰ | R⁵¹ |
|---|---|---|---|---|---|---|
| | R⁵⁴ | R⁵⁶ | B⁰ | V⁰ | | |
| 4-(Tetrazol-1-yl) | 2-F | H | O | CH | C(O)O | t-Butyl |
| | - | H | CH₂ | N | | |
| 4-SO₂CH₃ | 2-F | CH₃ | O | N | C(O)O | t-Butyl |
| | 3-F | CH₃ | CH₂ | CH | | |
| 4-SO₂CH₃ | - | CH₃ | S | N | C(O) | Isopropyl |
| | - | H | CH₂ | CH | | |
| 4- (1, 2, 4-Triazol-1-yl) | 2-Cl | H | O | CH | C(O)O | n-Butyl |
| | - | H | CH₂ | N | | |
| 4-(Tetrazol-1-yl) | 2-F | CH₃ | O | CH | C(O)O | Ethyl |
| | - | H | CH₂ | N | | |
| 4-(Tetrazol-1-yl) | | H | CH₂ | N | C(O)O | Cyclopropyl |
| | - | H | CH₂ | CH | | |
| 4- (1, 2, 4-Triazol-1-yl) | 2-F | CH₃ | 0 | N | C(O)O | t-Butyl |
| | - | CH₃ | CH₂ | CH | | |
| 4-SO₂NH₂ | - | H | O | CH | C(O)O | Isopropyl |
| | - | H | CH₂ | N | | |
| 4-SO₂-Phenyl | | H | CH₂ | N | C(O)O | Isobutyl |
| | - | H | O | CH | | |
| 4-SO₂CH₃ | 3-CF₃ | H | O | N | C(O) | Cyclopropyl |
| | - | H | C (CH₃) H | CH | | |
| 4-SO₂CH₃ | - | Cl | O | CH | C(O)O | t-Butyl |
| | - | - | CH₂ | N | | |

**TABLE 7**

| R⁵² | R⁵³ | R⁵⁵ | A⁰ | U⁰ | Q⁰ | R⁵¹ |
|---|---|---|---|---|---|---|
| | R⁵⁴ | R⁵⁶ | B⁰ | V⁰ | | |
| 4-(Tetrazol-1-yl) | 2-F | H | O | CH | 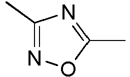 | Isopropyl |
| | - | H | CH₂ | N | | |
| 4-SO₂CH₃ | 2-F | CH₃ | O | N | 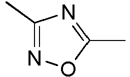 | Isopropyl |
| | 3-F | CH₃ | CH₂ | CH | | |
| 4- (1, 2, 4-Triazol-1-yl) | - | CH₃ | O | N | 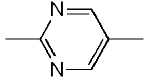 | Ethyl |
| | - | H | CH₂ | CH | | |
| 4-SO₂CH₃ | - | H | O | N | 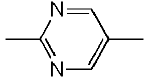 | Ethyl |
| | - | H | CH₂ | CH | | |

### Representative compound (4)

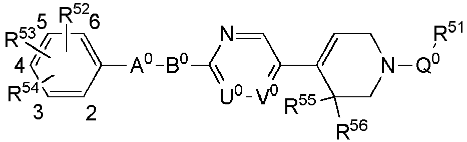

In the formula, R⁵², R⁵³, R⁵⁴, R⁵⁵, R⁵⁶, A⁰, B⁰, U⁰, V⁰, Q⁰, and R⁵¹ are set forth in Tables 8 and 9.

**TABLE 8**

| R⁵² | R⁵³ | R⁵⁵ | A⁰ | U⁰ | Q⁰ | R⁵¹ |
|---|---|---|---|---|---|---|
| | R⁵⁴ | R⁵⁶ | B⁰ | V⁰ | | |
| 4- (1, 2, 4-Triazol-1-yl) | - | CH₃ | O | CH | C(O)O | t-Butyl |
| | - | H | CH₂ | N | | |
| 4-(Tetrazol-1-yl) | 2-F | CH₃ | O | CH | C(O)O | t-Butyl |
| | - | H | CH₂ | N | | |
| 4-SO₂CH₃ | - | CH₃ | CH₂ | N | C(O) | Isobutyl |
| | - | CH₃ | CH₂ | CH | | |
| 4-SO₂CH₃ | 2-Cl | CH₃ | O | CH | C(O)O | n-Butyl |
| | - | H | CH₂ | N | | |
| 4-(Tetrazol-1-yl) | 2-F | CH₃ | O | CH | C(O)O | Ethyl |
| | 3-F | H | CH₂ | N | | |
| 4-SO₂CH₃ | 3-F | H | O | CH | C(O)O | Isopropyl |
| | 5-F | H | CH₂ | N | | |
| 4-SO₂NH₂ | - | H | O | N | C(O)O | t-Butyl |
| | - | H | CH₂ | CH | | |
| 4- (1, 2, 4-Triazol-1-yl) | 3-F | CH₃ | O | CH | C(O)O | Isopropyl |
| | - | H | CH₂ | N | | |

**TABLE 9**

| R⁵² | R⁵³ | R⁵⁵ | A⁰ | U⁰ | Q⁰ | R⁵¹ |
|---|---|---|---|---|---|---|
| | R⁵⁴ | R⁵⁶ | B⁰ | V⁰ | | |
| 4-SO₂CH₃ | 2-Br | H | O | N | 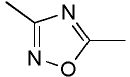 | Isopropyl |
| | - | H | CH₂ | CH | | |
| 4-(Tetrazol-1-yl) | 2-F | CH₃ | O | CH | 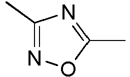 | Isopropyl |
| | - | H | CH₂ | N | | |
| 4- (1, 2, 4-Triazol-1-yl) | - | CH₃ | O | N | 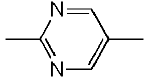 | Ethyl |
| | - | CH₃ | CH₂ | CH | | |
| 4-SO₂CH₃ | 2-F | CH₃ | O | CH | 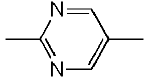 | Ethyl |
| | 3-F | H | CH₂ | N | | |

### Representative compound (5)

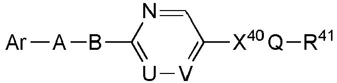

In the formula, Ar, A, B, U, V, X⁴⁰, Q⁰, and R⁴¹ are set forth in Tables 10 and 11.

**TABLE 10**

| Ar | A | U | X⁴⁰ | Q | R⁴¹ |
|---|---|---|---|---|---|
| | B | V | | | |
| 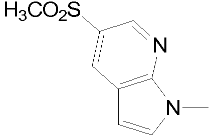 | CH₂ | CH | 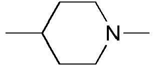 | C(O)O | Isopropyl |
| | Bond | N | | | |
| 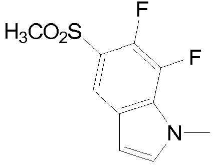 | CH₂ | CH | 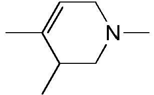 | 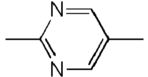 | Ethyl |
| | Bond | N | | | |
| 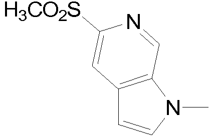 | CH₂ | N | 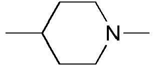 | C(O)O | t-Butyl |
| | Bond | CH | | | |
| 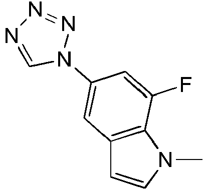 | CH₂ | CH | 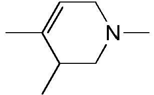 | C(O)O | t-Butyl |
| | Bond | N | | | |
| 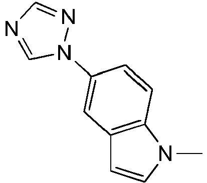 | CH₂ | N | 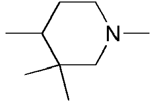 | 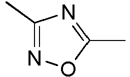 | Isopropyl |
| | Bond | CH | | | |

**TABLE 11**

| Ar | A | U | X⁴⁰ | Q | R⁴¹ |
|---|---|---|---|---|---|
| | B | V | | | |
| 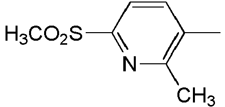 | O | N | 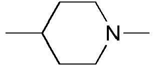 | C(O)O | Isopropyl |
| | CH₂ | CH | | | |
| 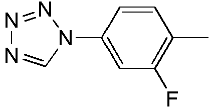 | O | CH | 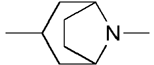 | C(O)O | t-Butyl |
| | CH₂ | N | | | |
| 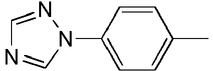 | O | CH | 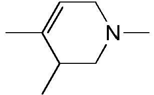 | 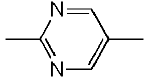 | Ethyl |
| | CH₂ | N | | | |
| 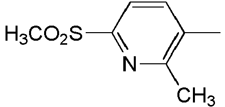 | O | CH | 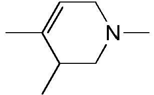 | C(O)O | t-Butyl |
| | CH₂ | N | | | |
| 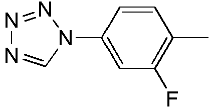 | CH₂ | N | 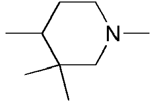 | C(O)O | t-Butyl |
| | CH₂ | CH | | | |

The pharmacological tests are described below.

### (Pharmacological test 1)

The GPR119 agonist effect is studied by measuring the effect of an analyte on increase of intracellular amount of cAMP in human GPR119 introduced cells. The testing method is described below.

### (1) Construction of the stable cell line expressing human GPR119

Human GPR119 gene (NM 178471) is purchased from ATCC (ATCC No. 10807349), and is amplyfied according to PCR to form BamHI site at 5' side and Apa I site at 3' side. The forward side primer is tcctggatccatggaatcatctttctcatt (sequence No. 1), and the reverse side primer is tcctgggcccttagccatcaaactctgagc (sequence No. 2). The PCR conditions are described below.

The double-stranded DNA is thermally denatured using a DNA polymerase (KOD-Plus-Ver. 2; TOYOBO #KOD-211) at 98°C for 10 seconds in one cycle. The denatured single-stranded DNA is annealed with the primers at 55°C for 30 seconds. The DNA is subjected to an extension reaction at 68°C for 1 minute and 15 seconds. The above-mentioned steps are repeated in 35 cycles. The PCR product is inserted into pcDNA5/FRT/TO (Invitrogen #V6520-20) plasmid. Flp-in T-Rex-293 cells (Invitorogen #R78007) are transfected with the obtained plasmid. The method of transfection is conducted in accordance with the protocol of the product.

### (2) Measurement of intracellular cAMP

The stable cell line expressing human GPR119 prepared in the above-mentioned method is plated on a 96-well plate at the concentration of 2,500 cells/well using Dulbecco's Modified Eagle Medium (DMEM) containing 10% fetal bovine serum (FBS). Twenty-four hours after plating, tetracyclin (Invitrogen #Q10019) is added at the final concentration of 20 ng/mL to induce hGPR119 gene expression. Twenty-four hours after, the medium is removed, and the cells are stimulated with an assay buffer (0.5 mM IBMX PBS(-)) containing the test compound at 37°C for 30 minutes. The amount of the intracellular cAMP is measured using a commercially available kit (HitHunter™ cAMP XS+ Assay: GE Healthcare #90007503) and a reader (FLUOstar Optima: BMG LABTECH). The test compound is dissolved in 100% DMSO, and added at the final concentration of 1%.

### (3) Experimental results

As is evident from Table 12 of Example 61 described below, the compounds of the present invention described in Examples 9, 20, 21, or the like show an excellent GPR119 agonist effect.

### (Pharmacological test 2)

As is also evident from Table 13 of Example 62 (Pharmacological test 2) described below, the compounds of the present invention described in Examples 28, 34, 42, 51, or the like show an excellent GPR119 agonist effect.

### (Pharmacological test 3)

Oral glucose tolerance is tested in normal mice.

### (1) Experimental procedure

In this experiment, the inhibitory effect of a test compound on glycemic excursions is examined after glucose administration in normal mice. The test methods are described below.

Male 9-week-old ICR mice, habituated to the experimental environment for two weeks, are fasted for 18 hours and used to this experiment. Mice are orally administered the analyte or vehicle (polyethylene glycol 400: ethanol: Tween 80 = 8:1:1). After 30 minutes, they were orally given glucose at the dose of 3 g/kg.

Blood is collected at just before the analyte or vehicle administration (-30 minutes), immediately before glucose challenge (0 minute), 20 minutes, 40 minutes, 60 minutes, and 120 minutes after glucose ingestion to determine blood glucose levels.

Inhibition rate (%) of the test compound versus vehicle in areas under the glycemic excursion curve between 0 minute and 120 minutes after glucose challenge is determined.

### (2) Experimental results

As is evident from Table 14 of Example 63 described below, the compounds of the present invention described in Example 9 show an excellent inhibitory effect on glycemic excursions.

As is described above, the compound represented by the formula (I) or (II), or a pharmaceutically acceptable salt thereof has a GPR119 agonist effect and an inhibitory effect on glycemic excursions. Therefore, they are expected to be used for treatment of diabetes. They are also expected to be used for a life-style related diseases such as obesity and metabolic syndrome.

The compound represented by the formula (I) or (II), or a pharmaceutically acceptable salt thereof can be used in combination with a conventional agent for treatment of diabetes.

The compound represented by the formula (I) or (II), or a pharmaceutically acceptable salt thereof can be administered to human beings by suitable administration methods such as oral administration or parenteral administration. It can also be used in combination with another agent for treatment of diabetes.

The compound or salt can be granulated in suitable manners for the preparation of pharmaceuticals. For instance, the compound or salt can be processed to give tablets, granule, powder, capsule, suspension, injection, suppository, and the like.

For the preparation of these pharmaceuticals, when they are tablets, appropriate additives such as excipients, disintegrators, binders, lubricants and dyes can be used. Lactose, D-mannitol, crystalline cellulose and glucose can be used as the excipients. Starch and carboxymethylcellulose calcium (CMC-Ca) can be used as the disintegrators, magnesium stearate, and talc as the lubricants. Hydroxypropylcellulose (HPC), gelatin and polyvinylpyrrolidone (PVP) can be used as the binders. For the preparation of injection, a solvent, a stabilizer, a solubilizer, a suspending agent, an emulsifier, an analgesic, a buffer, and a preservative can be used.

The compound represented by the formula (I) or (II), or a pharmaceutically acceptable salt thereof can be administered to an adult generally in an amount of 0.01 mg to 100 mg a day by injection and 1 mg to 2,000 mg a day by oral administration. The dosage can be adjusted according to age and conditions of the patient.

The invention is further described by the following non-limiting examples.

### [Example 1]

### tert-Butyl 4-[2-(4-methanesulfonylphenoxymethyl)pyrimidin-5-yl]piperidine-1-carboxylate

### (1) 2-Trimethylsilylethyl 4-(2-hydroxymethylpyrimidin-5-yl)-3,6-dihydro-2H-pyridine-1-carboxylate

To a solution of 5-bromo-2-hydroxymethylpyrimidine (50 mg, 0.265 mmol) and 2-trimethylsilylethyl 4-(4,4,5,5-tetramethyl-1,3,2-dioxaborolan-2-yl)-3,6-dihydro-2H-pyridine-1-carboxylate (103 mg, 0.291 mmol) in dry N,N-dimethylformamide (2 mL) was added [1,1'-bis(diphenylphosphino)ferrocene]dichloropalladium(II) dichloromethane adduct (13 mg, 15.9 µmol) and cesium carbonate (129 mg, 0.397 mmol). After stirring at 80°C overnight, cooled to room temperature, the reaction mixture was added water, and was extracted with ethyl acetate. The organic layer was washed with brine, dried over anhydrous sodium sulfate, and the solvent was removed under reduced pressure. The residue was purified by silica gel column chromatography (hexane/ethyl acetate = 1/2), to give the title compound (40 mg, yield 45%).
¹H NMR (CDCl₃, 400 MHz): δ= 0.06 (9H, s), 1.0-1.1 (2H, m), 2.54 (2H, br s), 3.60 (1H, br s), 3.73 (2H, t, J = 5 Hz), 4.1-4.3 (4H, m), 4.84 (2H, s), 6.18 (1H, br s), 8.72 (2H, s).

### (2) 2-Trimethylsilylethyl 4-(2-hydroxymethylpyrimidin-5-yl)piperidine-1-carboxylate

To a solution of 2-trimethylsilylethyl 4-(2-hydroxymethylpyrimidin-5-yl)-3,6-dihydro-2H-pyridine-1-carboxylate (40 mg, 0.119 mmol) in methanol (1 mL) was added 10% palladium-carbon (4 mg) and then the mixture was hydrogenated at room temperature for 3 hours under 1 atm of H₂. The reaction mixture was filtered through a celite pad, and the residue was purified by silica gel column chromatography (hexane/ethyl acetate = 1/2), to give the title compound (25 mg, yield 62%).
¹H NMR (CDCl₃, 400 MHz): δ= 0.06 (9H, s), 0.9-1.1 (2H, m), 1.5-1.7 (2H, m), 1.8-2.0 (2H, m), 2.6-3.0 (3H, m), 3.61 (1H, t, J = 5 Hz), 4.1-4.5 (4H, m), 4.83 (2H, d, J = 5 Hz), 8.59 (2H, s).

### (3) 2-Trimethylsilylethyl 4-[2-(4-methanesulfonylphenoxymethyl)pyrimidin-5-yl]piperidine-1-carboxylate

2-Trimethylsilylethyl 4-(2-hydroxymethylpyrimidin-5-yl)piperidine-1-carboxylate (25 mg, 74.1 µmol), 4-methanesulfonylphenol (19 mg, 0.111 mmol) and triphenylphosphine (29 mg, 0.111 mmol) was dissolved in dry tetrahydrofuran (1 mL). The mixture was cooled to 0°C, and then added 2.2 mol/L toluene solution of diethyl azodicarboxylate (51 µL, 0.111 mmol). After stirring at room temperature overnight, the reaction mixture was poured into water, extracted with ethyl acetate, the organic layer was washed with brine, dried over anhydrous sodium sulfate, and the solvent was removed under reduced pressure. The residue was purified by silica gel column chromatography (chloroform/methanol = 98/2 and hexane/ethyl acetate = 2/3), to give the title compound (17 mg, yield 46%) .
¹H NMR (CDCl₃, 400 MHz): δ= 0.05 (9H, s), 0.9-1.1 (2H, m), 1.6-1.8 (2H, m), 1.8-2.0 (2H, m), 2.6-3.0 (3H, m), 3.02 (3H, s), 4.1-4.5 (4H, m), 5.36 (2H, s), 7.14 (2H, d, J = 9 Hz), 7.86 (2H, d, J = 9 Hz), 8.63 (2H, s).

### (4) tert-Butyl 4-[2-(4-methanesulfonylphenoxymethyl)pyrimidin-5-yl]piperidine-1-carboxylate

To a solution of 2-trimethylsilylethyl 4-[2-(4-methanesulfonylphenoxymethyl)pyrimidin-5-yl]piperidine-1-carboxylate (16 mg, 32.5 µmol) in dry tetrahydrofuran (0.5 mL) was added 1.0 M tetrabutylammonium fluoride-tetrahydrofuran solution (49 µL, 48.8 µmol), and the mix-tuire was stirred at room temperature overnight. The reaction mixture was added chloroform, the organic layer was washed with brine, dried over anhydrous sodium sulfate, and the solvent was removed under reduced pressure.

To a solution of the resulting mixture in tetrahydrofuran (0.5 mL) - water (0.1 mL) was added di-tert-butyl dicarbonate (11 mg, 48.8 µmol) in dry tetrahydrofuran (0.3 mL), and stirred at room temperature for 1.5 hours. The reaction mixture was poured into saturated aqueous sodium hydrogen carbonate solution, and was extracted with ethyl acetate. The organic layer was washed with brine, dried over anhydrous sodium sulfate, and the solvent was removed under reduced pressure. The residue was purified by silica gel column chromatography (hexane/ethyl acetate = 2/3 → 1/2), to give the title compound as a white crystal (11 mg, yield 77%).
¹H NMR (CDCl₃, 400 MHz): δ= 1.48 (9H, s), 1.6-1.8 (2H, m), 1.8-2.0 (2H, m), 2.6-2.9 (3H, m), 3.02 (3H, s), 4.2-4.4 (2H, m), 5.36 (2H, s), 7.14 (2H, d, J = 9 Hz), 7.86 (2H, d, J = 9 Hz), 8.63 (2H, s).

### [Example 2]

### tert-Butyl 4-[5-(4-methanesulfonylphenoxymethyl)pyrazin-2-yl]-3,6-dihydro-2H-pyridine-1-carboxylate

### (1) 2-Chloro-5-(4-methanesulfonylphenoxymethyl)pyrazine

4-methanesulfonylphenol (211 mg, 1.23 mmol) was dissolved in N,N-dimethylformamide (2 mL). Under cooling in ice-bath, to the mixture was added 60% sodium hydride (49 mg, 1.23 mmol), and stirred for 30 minutes. To the mixture was added 2-chloro-5-chloromethylpyrazine (200 mg, 1.23 mmol), and the mixture was stirred at room temperature for 3 hours. The reaction mixture was poured into water, and was extracted with ethyl acetate. The organic layer was washed with brine, dried over anhydrous sodium sulfate, and the solvent was removed under reduced pressure. The residue was purified by silica gel column chromatography (hexane/ethyl acetate = 1/1), to give the title compound (273 mg, yield 74%).
¹H NMR (CDCl₃, 400 MHz): δ= 3.04 (3H, s), 5.30 (2H, s), 7.14 (2H, d, J = 9 Hz), 7.91 (2H, d, J = 9 Hz), 8.59 (1H, s), 8.60 (1H, d, J = 1 Hz).

### (2) tert-Butyl 4-[5-(4-methanesulfonylphenoxymethyl)pyrazin-2-yl]-3,6-dihydro-2H-pyridine-1-carboxylate

The title compound was prepared from 2-chloro-5-(4-methanesulfonylphenoxymethyl)pyrazine (100 mg, 0.335 mmol) and tert-butyl 4-(4,4,5,5-tetramethyl-1,3,2-dioxaborolan-2-yl)-3,6-dihydro-2H-pyridine-1-carboxylate (103 mg, 0.335 mmol) by the similar manner as described in Example 1(1) as a white crystal (48 mg, yield 32%).
¹H NMR (CDCl₃, 400 MHz): δ= 1.50 (9H, s), 2.6-2.7 (2H, m), 3.03 (3H, s), 3.6-3.7 (2H, m), 4.1-4.2 (2H, m), 5.30 (2H, s), 6.72 (1H, br s), 7.14 (2H, d, J = 9 Hz), 7.89 (2H, d, J = 9 Hz), 8.69 (2H, s).

### [Example 3]

### tert-Butyl 4-[5-(4-methanesulfonylphenoxymethyl)pyrazin-2-yl]piperidine-1-carboxylate

The title compound was prepared from tert-butyl 4-[5-(4-methanesulfonylphenoxymethyl)pyrazin-2-yl]-3,6-dihydro-2H-pyridine-1-carboxylate (Example 2) (44 mg, 98.8 µmol) by the similar manner as described in Example 1(2) as a white crystal (36 mg, yield 82%).
¹H NMR (CDCl₃, 400 MHz): δ= 1.48 (9H, s), 1.7-2.0 (4H, m), 2.7-3.0 (3H, m), 3.04 (3H, s), 4.1-4.4 (2H, m), 5.28 (2H, s), 7.14 (2H, d, J = 9 Hz), 7.90 (2H, d, J = 9 Hz), 8.48 (1H, d, J = 1 Hz), 8.70 (1H, s).

### [Example 4]

### tert-Butyl 4-[2-(5-methanesulfonylindol-1-ylmethyl)pyrimidin-5-yl]piperidine-1-carboxylate

### (1) 2-Trimethylsilylethyl 4-[2-(methanesulfonyloxymethyl)pyrimidin-5-yl]piperidine-1-carboxylate

To a solution of 2-trimethylsilylethyl 4-[2-(hydroxymethyl)pyrimidin-5-yl]piperidine-1-carboxylate (Example 1(2)) (26 mg, 74.1 µmol) in dichloromethane (0.7 mL) was added triethylamine (15.5 µL, 0.111 mmol) under ice-cooling. To this was added dropwise a solution of methanesulfonyl chloride (7 µL, 88.9 µmol) in dichloromethane (0.5 mL). After stirring at room temperature for 1 hour, the solvent was removed under reduced pressure, to give the crude title compound as a red oil (31 mg).

### (2) 2-Trimethylsilylethyl 4-[2-(5-methanesulfonylindol-1-ylmethyl)pyrimidin-5-yl]piperidine-1-carboxylate

Above crude 2-trimethylsilylethyl 4-[2-(methanesulfonyloxymethyl)pyrimidin-5-yl]piperidine-1-carboxylate (31 mg), 5-methanesulfonylindol (12 mg, 62.2 µmol), potassium hydroxide (3.5 mg, 62.2 µmol), potassium iodide (15.5 mg, 93.2 µmol) and 18-crown-6-ether (16 mg, 62.2 µmol) was dissolved in toluene (0.6 mL). The mixture was stirred at 80°C for 15 hours, then, cooled to room temperature. The reaction mixture was added water, and was extracted with ethyl acetate. The organic layer was washed with brine, dried over anhydrous sodium sulfate, and the solvent was removed under reduced pressure. The residue was purified by silica gel column chromatography (hexane/ethyl acetate = 7/1 → 0/100), to give the title compound as a pale yellow oil (31 mg, yield 81% (2 steps)).
¹H NMR (CDCl₃, 400 MHz): δ= 0.04 (9H, s), 0.9-1.1 1 (2H, m), 1,5-1.7 (2H, m), 1.7-1.9 (2H, m), 2.6-2.8 (1H, m), 2.8-2.9 (2H, m), 3.05 (3H, s), 4.1-4.3 (2H, m), 4.2-4.4 (2H, m), 5.54 (2H, s), 6.72 (1H, dd, J = 1 Hz,2 Hz), 7.45 (1H, d, J = 2 Hz), 7.53 (1H, d, J = 9 Hz), 7.70 (1H, dd, J = 2 Hz, 9 Hz), 8.27 (1H, d, J = 1 Hz), 8.53 (2H, s).

### (3) tert-Butyl 4-[2-(5-methanesulfonylindol-1-ylmethyl)pyrimidin-5-yl]piperidine-1-carboxylate

The title compound was prepared from 2-trimethylsilylethyl 4-[2-(5-methanesulfonylindol-1-ylmethyl)pyrimidin-5-yl]piperidine-1-carboxylate (31 mg, 60.0 µmol) by the similar manner as described in Example 1(4) as a pale yellow amorphous (23 mg, yield 81%).
FAB-MS(m/z): 471 (M+1)
¹H NMR (CDCl₃, 400 MHz): δ= 1.47 (9H, s), 1.5-1.7 (2H, m), 1.7-1.9 (2H, m), 2.6-2.7 (1H, m), 2.7-2.9 (2H, m), 3.05 (3H, s), 4.2-4.3 (2H, m), 5.54 (2H, s), 6.72 (1H, d, J = 2 Hz), 7.45 (1H, d, J = 2 Hz), 7.54 (1H, d, J = 8 Hz), 7.71 (1H, dd, J = 2 Hz, 8 Hz), 8.27 (1H, d, J = 2 Hz), 8.53 (2H, s).

### [Example 5]

### tert-Butyl 4-[2-(2-fluoro-4-nitrophenoxymethyl)pyrimidin-5-yl]piperidine-1-carboxylate

### (1) 2-Trimethylsilylethyl 4-[2-(2-fluoro-4-nitrophenoxymethyl)pyrimidin-5-yl]piperidine-1-carboxylate

The title compound was prepared from 2-trimethylsilylethyl 4-(2-hydroxymethylpyrimidin-5-yl)piperidine-1-carboxylate (25 mg, 0.130 mmol) (Example 1(2)) and 2-fluoro-4-nitrophenol (20 mg, 0.130 mmol) by the similar manner as described in Example 1(3) as a colorless oil (33 mg, yield 93%).
¹H NMR (CDCl₃, 400 MHz): δ= 0.05 (9H, s), 1.02 (2H, t, J = 8 Hz), 1.6-1.8 (2H, m), 1.8-1.9 (2H, m), 2.7-2.8 (1H, m), 2.8-3.0 (2H, m), 4.21 (2H, t, J = 8 Hz), 4.3-4.4 (2H, m), 5.45 (2H, s), 7.10 (1H, t, J = 8 Hz), 7.9-8.1 (2H, m), 8.63 (2H, s).

### (2) tert-Butyl 4-[2-(2-fluoro-4-nitrophenoxymethyl)pyrimidin-5-yl]piperidine-1-carboxylate

The title compound was prepared from 2-trimethylsilylethyl 4-[2-(2-fluoro-4-nitrophenoxymethyl)pyrimidin-5-yl]piperidine-1-carboxylate (33 mg, 69.2 µmol) by the similar manner as described in Example 1(4) as a pale yellow oil (28 mg, yield 94%).
¹H NMR (CDCl₃, 400 MHz): δ= 1.50 (9H, s), 1.6-1.8 (2H, m), 1.8-1.9 (2H, m), 2.7-2.8 (1H, m), 2.8-3.0 (2H, m), 4.3-4.4 (2H, m), 5.45 (2H, s), 7.10 (1H, t, J = 8 Hz), 7.9-8.1 (2H, m), 8.63 (2H, s).

### [Example 6]

### tert-Butyl 4-[2-(4-amino-2-fluorophenoxymethyl)pyrimidin-5-yl]piperidine-1-carboxylate

A suspension of zinc powder (154 mg, 2.36 mmol) in 0.5 mol/L hydrogen chloride (2 mL) was stirred at room temperature for 5 mintues, and then filtered. After washing by water and ethanol, the zinc solid was added to a solution of calcium chloride (7 mg, 64.8 µmol) in water (2 mL)-ethanol (2 mL), and then warmed to 90°C. To this was added a solution of tert-butyl 4-[2-(2-fluoro-4-nitrophenoxymethyl)pyrimidin-5-yl]piperidine-1-carboxylate (Example 5) (28 mg, 64.8 µmol) in ethanol (1 mL). After stirring at 90°C for 1 hour, the mixture was cooled to room temperature and the insoluble material was filtered off. The filtrate was concentrated to dryness, and the residue was diluted with ethyl acetate, the organic layer was dried over anhydrous sodium sulfate. The solvent was removed under reduced pressure, to give the title compound as a brown oil (22 mg, yield 84%).
¹H NMR (CDCl₃, 400 MHz): δ= 1.49 (9H, s), 1.6-1.8 (2H, m), 1.8-1.9 (2H, m), 2.6-2.9 (3H, m), 4.2-4.4 (2H, m), 5.23 (2H, s), 6.3-6.4 (1H, m), 6.46 (1H, dd, J = 2 Hz,12 Hz), 6.89 (1H, t, J = 9 Hz), 8.63 (2H, s).

### [Example 7]

### tert-Butyl 4-[2-[2-fluoro-4-(tetrazol-1-yl)phenoxymethyl]pyrimidin-5-yl]piperidine-1-carboxylate

To a solution of tert-butyl 4-[2-(4-amino-2-fluorophenoxymethyl)pyrimidin-5-yl]piperidine-1-carboxylate (Example 6) (22 mg, 54.7 µmol) in acetic acid (1 mL) was added triethyl orthoformate (50 µL, 0.301 mmol) and sodium azide (16 mg, 0.246 mmol), and the mixture was warmed up to 90°C. After stirring at the same temperature for an additional 3 hours, then cooled, the reaction mixture was added water and saturated aqueous sodium hydrogen carbonate solution, and was extracted with ethyl acetate. The organic layer was washed with brine, dried over anhydrous sodium sulfate, and the solvent was removed under reduced pressure. The residue was purified by silica gel column chromatography (hexane/ethyl acetate = 7/1 → 0/100), and recrystallized from ethyl acetate and hexane to give the title compound as a pale yellow crystal (7 mg, yield 28%).
FAB-MS (m/z) : 456 (M+1)
¹H NMR (CDCl₃, 400 MHz): δ= 1.48 (9H, s), 1.6-1.8 (2H, m), 1.8-1.9 (2H, m), 2.6-2.9 (3H, m), 4.2-4.4 (2H, m), 5.42 (2H, s), 7.22 (1H, t, J = 9 Hz), 7.38 (1H, td, J = 2 Hz, 9 Hz), 7.51 (1H, dd, J = 2 Hz, 11 Hz), 8.64 (2H, s), 8.89 (1H, s).

### [Example 8]

### tert-Butyl 4-[5-(5-methanesulfonylindol-1-ylmethyl)pyrazin-2-yl]-3,6-dihydro-2H-pyridine-1-carboxylate

### (1) 2-Chloro-5-(5-methanesulfonylindol-1-ylmethyl)pyrazine

The title compound was prepared from 2-chloro-5-chloromethylpyrazine (105 mg, 0.64 mmol) and 5-methanesulufonylindole (126 mg, 0.64 mmol) by the similar manner as described in Example 4(2) as a pale yellow amorphous (86 mg, yield 42%).
¹H NMR (CDCl₃, 400 MHz): δ= 3.06 (3H, s), 5.50 (2H, s), 6.75 (1H, d, J = 3 Hz), 7.37 (1H, d, J = 3 Hz), 7.44 (1H, d, J = 9 Hz), 7.74 (1H, dd, J = 2 Hz, 9 Hz), 8.0-8.1 (1H, m), 8.29 (1H, d, J = 2 Hz), 8.5-8.6 (1H, m).

### (2) tert-Butyl 4-[5-(5-methanesulfonylindol-1-ylmethyl)pyrazin-2-yl]-3,6-dihydro-2H-pyridine-1-carboxylate

The title compound was prepared from 2-chloro-5-(5-methanesulfonylindol-1-ylmethyl)pyrazine (86 mg, 0.27 mmol) and tert-butyl 4-(4,4,5,5-tetramethyl-1,3,2-dioxaborolan-2-yl)-3,6-dihydro-2H-pyridine-1-carboxylate (83 mg, 0.27 mmol) by the similar manner as described in Example 1(1) as a pale yellow amorphous (40 mg, yield 32%).
FAB-MS (m/z) : 469 (M+1)
¹H NMR (CDCl₃, 400 MHz): δ= 1.48 (9H, s), 2.5-2.7 (2H, m), 3.06 (3H, s), 3.6-3.7 (2H, m), 4.0-4.2 (2H, m), 5.50 (2H, s), 6.66 (1H, s), 6.74 (1H, d, J = 3 Hz), 7.39 (1H, d, J = 3 Hz), 7.47 (1H, d, J = 9 Hz), 7.73 (1H, dd, J = 2 Hz, 9 Hz), 8.1-8.2 (1H, m), 8.28 (1H, d, J = 2 Hz), 8.6-8.7 (1H, m).

### [Example 9]

### tert-Butyl 4-[5-(5-methanesulfonylindol-1-ylmethyl)pyrazin-2-yl]piperidine-1-carboxylate

The title compound was prepared from tert-butyl 4-[5-(5-methanesulfonylindol-1-ylmethyl)pyrazin-2-yl]-3,6-dihydro-2H-pyridine-1-carboxylate (Example 8) (28 mg, 60 µmol) by the similar manner as described in Example 1(2) as a white amorphous (8 mg, yield 28%).
FAB-MS(m/z): 471 (M+1)
¹H NMR (CDCl₃, 400 MHz): δ= 1.46 (9H, s), 1.6-1.8 (2H, m), 1.8-1.9 (2H, m), 2.7-2.9 (3H, m), 3.06 (3H, s), 4.1-4.4 (2H, m), 5.49 (2H, s), 6.74 (1H, d, J = 3 Hz), 7.39 (1H, d, J = 3 Hz), 7.49 (1H, d, J = 9 Hz), 7.74 (1H, dd, J = 2 Hz,9 Hz), 8.1-8.2 (1H, m), 8.29 (1H, d, J = 2 Hz), 8.4-8.5 (1H, m).

### [Example 10]

### tert-Butyl 3-[5-(4-methanesulfonylphenoxymethyl)pyrazin-2-yl]-8-azabicyclo[3.2.1]oct-2-ene-8-carboxylate

The title compound was prepared from 2-chloro-5-(4-methanesulfonylphenoxymethyl)pyrazine (100 mg, 0.335 mmol) and tert-butyl 3-(4,4,5,5-tetramethyl-1,3,2-dioxaborolan-2-yl)-8-azabicyclo[3.2.1]oct-2-ene-8-carboxylate (112 mg, 0.335 mmol) by the similar manner as described in Example 1(1) as a white crystal (36 mg, yield 22%).
¹H NMR (CDCl₃, 400 MHz): δ= 1.45 (9H, s), 1.6-1.8 (1H, m), 1.9-2.1 (2H, m), 2.2-2.4 (1H, m), 2.3-2.5 (1H, m), 2.8-3.3 (1H, m), 3.03 (3H, s), 4.4-4.7 (2H, m), 5.29 (2H, s), 7.07 (1H, br d), 7.12 (2H, d, J = 9 Hz), 7.88 (2H, d, J = 9 Hz), 8.66 (2H, s).

### [Example 11]

### tert-Butyl 3-[5-(4-methanesulfonylphenoxymethyl)pyrazin-2-yl]-8-azabicyclo[3.2.1]octane-8-carboxylate

The title compound was prepared from tert-Butyl 3-[5-(4-methanesulfonylphenoxymethyl)pyrazin-2-yl]-8-azabicyclo[3.2.1]oct-2-ene-8-carboxylate (Example 10)(25 mg, 53.0 µmol) by the similar manner as described in Example 1(2) as a white crystal (15 mg, yield 59%, diastereomer ratio = 3:7).
¹H NMR (CDCl₃, 400 MHz): δ= 1.49 (6.3H, s), 1.51 (2.7H, s), 1.4-2.1 (7.3H, m), 2.3-2.6 (0.7H, m), 3.04 (3H, s), 3.0-3.2 (0.3H, m), 3.2-3.4 (0.7H, m), 4.2-4.5 (2H, m), 5.27 (1.4H, s), 5.28 (0.6H, s), 7.1-7.2 (2H, m), 7.8-8.0 (2H, m), 8.46 (0.7H, d, J = 1 Hz), 8.57 (0.3H, d, J = 1 Hz), 8.6-8.7 (1H, m).

### [Example 12]

### tert-Butyl 4-[5-[2-fluoro-4-(tetrazol-1-yl)phenoxymethyl]pyrazin-2-yl]-3,6-dihydro-2H-pyridine-1-carboxylate

### (1) 2-Chloro-5-[2-fluoro-4-(tetrazol-1-yl)phenoxymethyl]pyrazine

To a solution of 2-fluoro-4-(tetrazol-1-yl)phenol (100 mg, 0.555 mmol) in ethanol (6 mL) was added 0.5M potassium hydroxide ethanol solution (1.2 mL, 0.600mmol), and stirred at room temperature for 40 minutes. The reaction mixture was concentrated under reduced pressure to give the crude potassium 2-fluoro-4-(tetrazol-1-yl)phenolate. To a solution of crude potassium salt in dimethylsulfoxide (6 mL) was added 2-Chloro-5-chloromethylpyrazine (101 mg, 0.617 mmol), and sttired at room temperature for 1 hour. The reaction mixture was poured into water, and was extracted with ethyl acetate. The organic layer was washed with brine, dried over anhydrous sodium sulfate, and the solvent was removed under reduced pressure. The residue was purified by silica gel column chromatography (chloroform/methanol = 100/1), to give the title compound as a pale brown crystal (134 mg, yield 79%).
¹H NMR (CDCl₃, 400 MHz): δ= 5.37 (2H, s), 7.2-7.3 (1H, m), 7.4-7.5 (1H, m), 7.56 (1H, dd, J = 2 Hz, 11 Hz), 8.61 (1H, br s), 8.66 (1H, br s), 8.92 (1H, s).

### (2) tert-Butyl 4-[5-[2-fluoro-4-(tetrazol-1-yl)phenoxymethyl]pyrazin-2-yl]-3,6-dihydro-2H-pyridine-1-carboxylate

The title compound was prepared from 2-Chloro-5-[2-fluoro-4-(tetrazol-1-yl)phenoxymethyl]pyrazine (50 mg, 0.163 mmol) and tert-butyl 4-(4,4,5,5-tetramethyl-1,3,2-dioxaborolan-2-yl)-3,6-dihydro-2H-pyridine-1-carboxylate (50 mg, 0.163 mmol) by the similar manner as described in Example 1(1) as a pale yellow amorphous (19 mg, yield 25%).
FAB-MS(m/z): 454 (M+1)
¹H NMR (CDCl₃, 400 MHz): δ= 1.48 (9H, s), 2.6-2.8 (2H, m), 3.6-3.8 (2H, m), 4.1-4.3 (2H, m), 5.37 (2H, s), 6.73 (1H, br s), 7.26 (1H, t, J = 9 Hz), 7.3-7.5 (1H, m), 7.54 (1H, dd, J = 2 Hz, 11 Hz), 8.70 (1H, d, J = 1 Hz), 8.76 (1H, br s), 8.92 (1H, s).

### [Example 13]

### tert-Butyl 4-[5-[4-(1,2,4-triazol-1-yl)phenoxymethyl]pyrazin-2-yl]-3,6-dihydro-2H-pyridine-1-carboxylate

### (1) 2-Chloro-5-[4-(1,2,4-triazol-1-yl)phenoxymethyl]pyrazine

The title compound was prepared from 4-(1,2,4-triazol-1-yl)phenol (100 mg, 0.621 mmol) by the similar manner as described in Example 2(1) as a pale brown amorphous (144 mg, yield 81%).
¹H NMR (DMSO-d₆, 400 MHz): δ= 5.30 (2H, s), 7.24 (2H, d, J = 9 Hz), 7.78 (2H, d, J = 9 Hz), 8.17 (1H, s), 8.70 (1H, s), 8.83 (1H, s), 9.17 (1H, s).

### (2) tert-Butyl 4-[5-[4-(1,2,4-triazol-1-yl)phenoxymethyl]pyrazin-2-yl]-3,6-dihydro-2H-pyridine-1-carboxylate

The title compound was prepared from 2-chloro-5-[4-(1,2,4-triazol-1-yl)phenoxymethyllpyrazine (70 mg, 0.243 mmol) and tert-butyl 4-(4,4,5,5-tetramethyl-1,3,2-dioxaborolan-2-yl)-3,6-dihydro-2H-pyridine-1-carboxylate (75 mg, 0.243 mmol) by the similar manner as described in Example 1(1) as a white crystal (54 mg, yield 51%).
FAB-MS(m/z): 435 (M+1)
m.p.152-154°C
¹H NMR (CDCl₃, 400 MHz): δ= 1.50 (9H, s), 2.6-2.8 (2H, m), 3.6-3.8 (2H, m), 4.1-4.3 (2H, m), 5.28 (2H, s), 6.71 (1H, br s), 7.12 (2H, d, J = 9 Hz), 7.60 (2H, d, J = 9 Hz), 8.08 (1H, s), 8.45 (1H, s), 8.69 (1H, s), 8.72 (1H, s). IR (KBr, cm⁻¹): 3122, 2976, 2929, 1685, 1651, 1525, 1483, 1448, 1410, 1394, 1363, 1300, 1277, 1255, 1236, 1273, 1155, 1117, 1066, 1030, 982, 958, 903, 858, 829, 674, 521.

### [Example 14]

### tert-Butyl 4-[5-(5-methanesulfonyl-1H-pyrrolo[2,3-blpyridin-1-ylmethyl)pyrazin-2-yl]-3,6-dihydro-2H-pyridine-1-carboxylate

### (1) 2-Chloro-5-(5-methanesulfonyl-1H-pyrrolo[2,3-b]pyridin-1-ylmethyl)pyrazine

The title compound was prepared from 2-chloro-5-chloromethylpyrazine (135 mg, 0.69 mmol) by the similar manner as described in Example 4(2) (142 mg, yield 64%).
¹H NMR (CDCl₃, 400 MHz): δ= 3.12 (3H, s), 5.67 (2H, s), 6.70 (1H, d, J = 3 Hz), 7.53 (1H, d, J = 3 Hz), 8.35 (1H, s), 8.49 (1H, d, J = 2 Hz), 8.53 (1H, d, J = 1 Hz), 8.86 (1H, d, J = 2 Hz).

### (2) tert-Butyl 4-[5-(5-methanesulfonyl-1H-pyrrolo[2,3-b]pyridin-1-ylmethyl)pyrazin-2-yl]-3,6-dihydro-2H-pyridine-1-carboxylate

The title compound was prepared from 2-chloro-5-(5-methanesulfonyl-1H-pyrrolo[2,3-b]pyridin-1-ylmethyl)pyrazine (30 mg, 0.093 mmol) and tert-butyl 4-(4,4,5,5-tetramethyl-1,3,2-dioxaborolan-2-yl)-3,6-dihydro-2H-pyridine-1-carboxylate (29 mg, 0.093 mmol) by the similar manner as described in Example 1(1) as a pale yellow amorphous (38 mg, yield 87%).
FAB-MS(m/z): 470 (M+1)
¹H NMR (CDCl₃, 400 MHz): δ= 1.48 (9H, s), 2.5-2.7 (2H, m), 3.12 (3H, s), 3.6-3.7 (2H, m), 4.0-4.2 (2H, m), 5.67 (2H, s), 6.65 (1H, br s), 6.68 (1H, d, J = 3 Hz), 7.54 (1H, d, J = 3 Hz), 8.46 (1H, s), 8.49 (1H, d, J = 2 Hz), 8.62 (1H, s), 8.86 (1H, d, J = 2 Hz).

### [Example 15]

### tert-Butyl 4-[5-(5-methanesulfonyl-1H-pyrrolo[2,3-b]pyridin-1-ylmethyl)pyrazin-2-yl]-3-methyl-3,6-dihydro-2H-pyridine-1-carboxylate

The title compound was prepared from 2-chloro-5-(5-methanesulfonyl-1H-pyrrolo[2,3-b]pyridin-1-ylmethyl)pyrazine (Example 14(1)) (30 mg, 0.093 mmol) and tert-butyl 3-methyl-4-(4,4,5,5-tetramethyl-1,3,2-dioxaborolan-2-yl)-3,6-dihydro-2H-pyridine-1-carboxylate (30 mg, 0.093 mmol) by the similar manner as described in Example 1(1) as a pale yellow amorphous (14 mg, yield 31%).
FAB-MS(m/z): 484 (M+1)
¹H NMR (CDCl₃, 400 MHz): δ= 1.06 (3H, d, J = 7 Hz), 1.48 (9H, s), 3.0-3.2 (2H, m), 3.12 (3H, s), 3.7-4.1 (2H, m), 4.3-4.7 (1H, m), 5.68 (2H, s), 6.48 (1H, br s), 6.69 (1H, d, J = 3 Hz), 7.55 (1H, d, J = 3 Hz), 8.46 (1H, s), 8.49 (1H, d, J = 2 Hz), 8.63 (1H, s), 8.87 (1H, d, J = 2 Hz).

### [Example 16]

### tert-Butyl 4-[5-(4,6-difluoro-5-methanesulfonylindol-1-ylmethyl)pyrazin-2-yl]-3,6-dihydro-2H-pyridine-1-carboxylate

### (1) 2-Chloro-5-(4,6-difluoro-5-methanesulfonylindol-1-ylmethyl)pyrazine

The title compound was prepared from 2-chloro-5-chloromethylpyrazine (106 mg, 0.65 mmol) and 4,6-difluoro-5-methanesulfonylindole (150 mg, 0.65 mmol) by the similar manner as described in Example 4(2) (28 mg, yield 12%).
¹H NMR (CDCl₃, 400 MHz): δ= 3.29 (3H, s), 5.41 (2H, s), 6.77 (1H, d, J = 3 Hz), 7.00 (1H, d, J = 11 Hz), 7.26 (1H, d, J = 3 Hz), 8.14 (1H, s), 8.55 (1H, d, J = 1 Hz).

### (2) tert-Butyl 4-[5-(4,6-difluoro-5-methanesulfonylindol-1-ylmethyl)pyrazin-2-yl]-3,6-dihydro-2H-pyridine-1-carboxylate

The title compound was prepared from 2-chloro-5-(4,6-difluoro-5-methanesulfonylindol-1-ylmethyl)pyrazine (28 mg, 0.078 mmol) and tert-butyl 4-(4,4,5,5-tetramethyl-1,3,2-dioxaborolan-2-yl)-3,6-dihydro-2H-pyridine-1-carboxylate (28 mg, 0.078 mmol) by the similar manner as described in Example 1(1) as a pale yellow amorphous (8 mg, yield 20%).
FAB-MS (m/z) : 505 (M+1)
¹H NMR (CDCl₃, 400 MHz): δ= 1.48 (9H, s), 2.5-2.7 (2H, m), 3.29 (3H, s), 3.6-3.7 (2H, m), 4.1-4.2 (2H, m), 5.39 (2H, s), 6.68 (1H, br s), 6.77 (1H, d, J = 3 Hz), 7.01 (1H, d, J = 11 Hz), 7.27 (1H, d, J = 3 Hz), 8.26 (1H, d, J = 1 Hz), 8.65 (1H, d, J = 1 Hz).

### [Example 17]

### tert-Butyl 3-methyl-4-[5-[4-(1,2,4-triazol-1-yl)phenoxymethyl]pyrazin-2-yl]-3,6-dihydro-2H-pyridine-1-carboxylate

The title compound was prepared from 2-chloro-5-[4-(1,2,4-triazol-1-yl)phenoxymethyllpyrazine (Example 13(1)) (72 mg, 0.250 mmol) and tert-butyl 3-methyl-4-(4,4,5,5-tetramethyl-1,3,2-dioxaborolan-2-yl)-3,6-dihydro-2H-pyridine-1-carboxylate (81 mg, 0.250 mmol) by the similar manner as described in Example 1(1) as a pale yellow amorphous (2.3 mg, yield 2%).
FAB-MS (m/z) : 449 (M+1)
¹H NMR (CDCl₃, 400 MHz): δ= 1.11 (3H, d, J = 7 Hz), 1.50 (9H, s), 3.0-3.3 (2H, m), 3.7-4.1 (2H, m), 4.2-4.7 (1H, m), 5.28 (2H, s), 6.54 (1H, br s), 7.13 (1H, d, J = 9 Hz), 7.60 (1H, d, J = 9 Hz), 8.08 (1H, s), 8.46 (1H, s), 8.70 (1H, s), 8.73 (1H, s).

### [Example 18]

### tert-Butyl 4-[5-(2,3-difluoro-4-methanesulfonylphenoxymethyl)pyrazin-2-yl]-3,6-dihydro-2H-pyridine-1-carboxylate

### (1) 2-Chloro-5-(2,3-difluoro-4-methanesulfonylphenoxymethyl)pyrazine

The title compound was prepared from 2,3-difluoro-4-methanesulfonylphenol (150 mg, 0.721 mmol) by the similar manner as described in Example 2(1) (165 mg, yield 68%).
¹H NMR (CDCl₃, 400 MHz): δ= 3.21 (3H, s), 5.37 (2H, s), 6.9-7.1 (1H, m), 7.6-7.8 (1H, m), 8.61 (1H, d, J = 1 Hz), 8.62 (1H, s).

### (2) tert-Butyl 4-[5-(2,3-difluoro-4-methanesulfonylphenoxymethyl)pyrazin-2-yl]-3,6-dihydro-2H-pyridine-1-carboxylate

The title compound was prepared from 2-chloro-5-(2,3-difluoro-4-methanesulfonylphenoxymethyl)pyrazine (50 mg, 0.149 mmol) and tert-butyl 4-(4,4,5,5-tetramethyl-1,3,2-dioxaborolan-2-yl)-3,6-dihydro-2H-pyridine-1-carboxylate (46 mg, 0.149 mmol) by the similar manner as described in Example 1(1) as a white crystal (44 mg, yield 61%).
FAB-MS(m/z): 482 (M+1)
m.p.176-179°C
¹H NMR (CDCl₃, 400 MHz): δ= 1.50 (9H, s), 2.6-2.8 (2H, m), 3.21 (3H, s), 3.6-3.8 (2H, m), 4.1-4.3 (2H, m), 5.37 (2H, s), 6.74 (1H, br s), 6.9-7.1 (1H, m), 7.6-7.8 (1H, m), 8.69 (1H, s), 8.72 (1H, s).
IR (KBr, cm⁻¹): 3008, 2976, 2929, 1689, 1618, 1512, 1464, 1412, 1396, 1365, 1306, 1238, 1169, 1130, 1095, 1026, 993, 897, 866, 812, 771, 528, 490.

### [Example 19]

### tert-Butyl 4-[5-(4,6-difluoro-5-methanesulfonylindol-1-ylmethyl)pyrazin-2-yl]-3-methyl-3,6-dihydro-2H-pyridine-1-carboxylate

The title compound was prepared from 2-chloro-5-(4,6-difluoro-5-methanesulfonylindol-1-ylmethyl)pyrazine(Example 16(1)) (25 mg, 0.08 mmol) and tert-butyl 3-methyl-4-(4,4,5,5-tetramethyl-1,3,2-dioxaborolan-2-yl)-3,6-dihydro-2H-pyridine-1-carboxylate (26 mg, 0.08 mmol) by the similar manner as described in Example 1(1) as a pale yellow amorphous (9 mg, yield 24%).
FAB-MS (m/z) : 519 (M+1)
¹H NMR (CDCl₃, 400 MHz): δ= 1.08 (3H, d, J = 7 Hz), 1.49 (9H, s), 3.0-3.2 (2H, m), 3.30 (3H, s), 3.7-4.1 (2H, m), 4.3-4.6 (1H, m), 5.39 (2H, s), 6.52 (1H, br s), 6.77 (1H, d, J = 3 Hz), 7.03 (1H, d, J = 11 Hz), 7.27 (1H, d, J = 3 Hz), 8.26 (1H, d, J = 1 Hz), 8.66 (1H, d, J = 1 Hz).

### [Example 20]

### tert-Butyl 4-[5-(6,7-difluoro-5-methanesulfonylindol-1-ylmethyl)pyrazin-2-yl]-3,6-dihydro-2H-pyridine-1-carboxylate

### (1) 2-Chloro-5-(6,7-difluoro-5-methanesulfonylindol-1-ylmethyl)pyrazine

The title compound was prepared from 2-chloro-5-chloromethylpyrazine (57 mg, 0.35 mmol) and 6,7-difluoro-5-methanesulfonylindole (82 mg, 0.35 mmol) by the similar manner as described in Example 4(2) as a pale yellow crystal (89 mg, yield 71%).
¹H NMR (CDCl₃, 400 MHz): δ= 3.23 (3H, s), 5.61 (2H, s), 6.71 (1H, d, J = 3 Hz), 7.34 (1H, d, J = 3 Hz), 7.99 (1H, d, J = 6 Hz), 8.20 (1H, s), 8.52 (1H, s).

### (2) tert-Butyl 4-[5-(6,7-difluoro-5-methanesulfonylindol-1-ylmethyl)pyrazin-2-yl]-3,6-dihydro-2H-pyridine-1-carboxylate

The title compound was prepared from 2-chloro-5-(6,7-difluoro-5-methanesulfonylindol-1-ylmethyl)pyrazine (30 mg, 0.084 mmol) and tert-butyl 4-(4,4,5,5-tetramethyl-1,3,2-dioxaborolan-2-yl)-3,6-dihydro-2H-pyridine-1-carboxylate (26 mg, 0.084 mmol) by the similar manner as described in Example 1(1) as a pale yellow amorphous (26 mg, yield 61%).
FAB-MS (m/z) : 505 (M+1)
¹H NMR (CDCl₃, 400 MHz): δ= 1.48 (9H, s), 2.5-2.7 (2H, m), 3.23 (3H, s), 3.6-3.7 (2H, m), 4.1-4.2 (2H, m), 5.60 (2H, s), 6.66 (1H, br s), 6.69 (1H, dd, J = 1 Hz,3 Hz), 7.34 (1H, d, J = 3 Hz), 8.00 (1H, d, J = 6 Hz), 8.31 (1H, s), 8.63 (1H, d, J = 1 Hz).

### [Example 21]

### tert-Butyl 4-[5-(6,7-difluoro-5-methanesulfonylindol-1-ylmethyl)pyrazin-2-yl]-3-methyl-3,6-dihydro-2H-pyridine-1-carboxylate

The title compound was prepared from 2-chloro-5-(6,7-difluoro-5-methanesulfonylindol-1-ylmethyl)pyrazine (Example 20(1)) (20 mg, 0.056 mmol) and tert-butyl 3-methyl-4-(4,4,5,5-tetramethyl-1,3,2-dioxaborolan-2-yl)-3,6-dihydro-2H-pyridine-1-carboxylate (18 mg, 0.056 mmol) by the similar manner as described in Example 1(1) as a pale yellow amorphous (3.7 mg, yield 13%).
FAB-MS (m/z) : 519 (M+1)
¹H NMR (CDCl₃, 400 MHz): δ= 1.07 (3H, d, J = 7 Hz), 1.49 (9H, s), 3.0-3.2 (2H, m), 3.23 (3H, s), 3.7-4.1 (2H, m), 4.3-4.6 (1H, m), 5.60 (2H, s), 6.50 (1H, br s), 6.71 (1H, t, J = 3 Hz), 7.34 (1H, d, J = 3 Hz), 8.01 (1H, d, J = 6 Hz), 8.32 (1H, s), 8.64 (1H, d, J = 1 Hz).

### [Example 22]

### tert-Butyl 4-[5-[5-(1,2,4-triazol-1-yl)indol-1-ylmethyl]pyrazin-2-yl]-3,6-dihydro-2H-pyridine-1-carboxylate

### (1) 2-Chloro-5-[5-(1,2,4-triazol-1-yl)indol-1-ylmethyl]pyrazine

The title compound was prepared from 2-chloro-5-chloromethylpyrazine (150 mg, 0.81 mmol) and 5-(1,2,4-triazol-1-yl)indole (133 mg, 0.81 mmol) by the similar manner as described in Example 4(2) as a pale yellow crystal (80 mg, yield 32%).
¹H NMR (CDCl₃, 400 MHz): δ= 5.48 (2H, s), 6.67 (1H, d, J = 3 Hz), 7.31 (1H, d, J = 3 Hz), 7.38 (1H, d, J = 9 Hz), 7.48 (1H, dd, J = 2 Hz, 9 Hz), 7.89 (1H, d, J = 2 Hz), 7.97 (1H, s), 8.10 (1H, s), 8.50 (1H, s), 8.55 (1H, s).

### (2) tert-Butyl 4-[5-[5-(1,2,4-triazol-1-yl)indol-1-ylmethyl]pyrazin-2-yl]-3,6-dihydro-2H-pyridine-1-carboxylate

The title compound was prepared from 2-chloro-5-[5-(1,2,4-triazol-1-yl)indol-1-ylmethyllpyrazine (25 mg, 0.08 mmol) and tert-butyl 4-(4,4,5,5-tetramethyl-1,3,2-dioxaborolan-2-yl)-3,6-dihydro-2H-pyridine-1-carboxylate (25 mg, 0.08 mmol) by the similar manner as described in Example 1(1) as a yellow amorphous (26 mg, yield 71%).
FAB-MS (m/z) : 458 (M+1)
¹H NMR (CDCl₃, 400 MHz): δ= 1.48 (9H, s), 2.5-2.7 (2H, m), 3.6-3.7 (2H, m), 4.1-4.2 (2H, m), 5.67 (2H, s), 6.65 (1H, br s), 6.68 (1H, d, J = 3 Hz), 7.34 (1H, d, J = 3 Hz), 7.41 (1H, d, J = 8 Hz), 7.47 (1H, dd, J = 2 Hz, 8 Hz), 7.88 (1H, d, J = 2 Hz), 8.10 (1H, s), 8.15 (1H, s), 8.50 (1H, s), 8.66 (1H, s).

### [Example 23]

### tert-Butyl 3-methyl-4-[5-[5-(1,2,4-triazol-1-yl)indol-1-ylmethyl]pyrazin-2-yl]-3,6-dihydro-2H-pyridine-1-carboxylate

The title compound was prepared from 2-chloro-[5-(1,2,4-triazol-1-yl)indol-1-ylmethyllpyrazine (Exam-ple22 (1)) (25 mg, 0.08 mmol) and tert-butyl 3-methyl-4-(4,4,5,5-tetramethyl-1,3,2-dioxaborolan-2-yl)-3,6-dihydro-2H-pyridine-1-carboxylate (26 mg, 0.08 mmol) by the similar manner as described in Example 1(1) as a white amorphous (9 mg, yield 24%).
FAB-MS(m/z): 472 (M+1)
¹H NMR (CDCl₃, 400 MHz): δ= 1.06 (3H, d, J = 7 Hz), 1.48 (9H, s), 3.0-3.2 (2H, m), 3.7-4.1 (2H, m), 4.1-4.6 (1H, m), 5.50 (2H, s), 6.49 (1H, br s), 6.68 (1H, d, J = 3 Hz), 7.35 (1H, d, J = 3 Hz), 7.43 (1H, d, J = 9 Hz), 7.48 (1H, dd, J = 2 Hz, 9 Hz), 7.89 (1H, d, J = 2 Hz), 8.10 (1H, s), 8.15 (1H, s), 8.50 (1H, s), 8.66 (1H, s).

### [Example 24]

### tert-Butyl 4-[2-(2,3-difluoro-4-methanesulfonylphenoxymethyl)pyrimidin-5-yl]-3-methyl-3,6-dihydro-2H-pyridine-1-carboxylate

### (1) 5-Bromo-2-(2,3-difluoro-4-methanesulfonylphenoxymethyl)pyrimidine

The title compound was prepared from 5-bromo-2-hydroxymethylpyrimidine (30 mg, 0.159 mmol) and 2,3-difluoro-4-methanesulfonylphenol (33 mg, 0.159 mmol) by the similar manner as described in Example 1(3) as a pale yellow crystal (34 mg, yield 56%).
¹H NMR (CDCl₃, 400 MHz): δ= 3.20 (3H, s), 5.42 (2H, s), 6.8-7.0 (1H, m), 7.5-7.7 (1H, m), 8.82 (2H, s).

### (2) tert-Butyl 4-[2-(2,3-difluoro-4-methanesulfonylphenoxymethyl)pyrimidin-5-yl]-3-methyl-3,6-dihydro-2H-pyridine-1-carboxylate

The title compound was prepared from 5-bromo-2-(2,3-difluoro-4-methanesulfonylphenoxymethyl)pyrimidine (34 mg, 89.7 µmol) and tert-butyl 3-methyl-4-(4,4,5,5-tetramethyl-1,3,2-dioxaborolan-2-yl)-3,6-dihydro-2H-pyridine-1-carboxylate (30 mg, 89.7 µmol) by the similar manner as described in Example 1(1) as a yellow amorphous (8 mg, yield 18%).
FAB-MS (m/z) : 496 (M+1)
¹H NMR (CDCl₃, 400 MHz): δ= 1.05 (3H, d, J = 7 Hz), 1.50 (9H, s), 2.7-2.9 (1H, m), 3.20 (3H, s), 3.33 (1H, dd, J = 4 Hz,13 Hz), 3.8-4.0 (1H, m), 3.86 (1H, dd, J = 3 Hz,13 Hz), 4.1-4.5 (1H, m), 5.46 (2H, s), 6.03 (1H, br s), 6.9-7.0 (1H, m), 7.5-7.7 (1H, m), 8.72 (2H, s).

### [Example 25]

### tert-Butyl 3-methyl-4-[2-[4-(1,2,4-triazol-1-yl)phenoxymethyl]pyrimidin-5-yl]-3,6-dihydro-2H-pyridine-1-carboxylate

### (1) tert-Butyl 4-(2-hydroxymethylpyrimidin-5-yl)-3-methyl-3,6-dihydro-2H-pyridine-1-carboxylate

The title compound was prepared from 5-bromo-2-hydroxymethylpyrimidine (175 mg, 0.928 mmol) and tert-butyl 3-methyl-4-(4,4,5,5-tetramethyl-1,3,2-dioxaborolan-2-yl)-3,6-dihydro-2H-pyridine-1-carboxylate (300 mg, 0.928 mmol) by the similar manner as described in Example 1(1) as a yellow oil (46 mg, yield 16%).
¹H NMR (CDCl₃, 400 MHz): δ= 1.04 (3H, d, J = 7 Hz), 1.50 (9H, s), 2.7-2.9 (1H, m), 3.34 (1H, dd, J = 4 Hz, 13 Hz), 3.57 (1H, t, J = 5 Hz), 3.8-4.0 (1H, m), 3.86 (1H, dd, J = 3 Hz,13 Hz), 4.1-4.5 (2H, m), 4.85 (2H, d, J = 5 Hz), 6.00 (1H, br s), 8.69 (2H, s).

### (2) tert-Butyl 3-methyl-4-[2-[4-(1,2,4-triazol-1-yl)phenoxymethyl]pyrimidin-5-yl]-3,6-dihydro-2H-pyridine-1-carboxylate

To a solution of tert-butyl 4-(2-hydroxymethylpyrimidin-5-yl)-3-methyl-3,6-dihydro-2H-pyridine-1-carboxylate (46 mg, 0.151 mmol) in dichloromethane (1 mL) was added triethylamine (30 µL, 0.226 mmol) under ice-cooling. To this was added dropwise a solution of methanesulfonyl chloride (14 µL, 0.181 mmol) in dichloromethane (0.5 mL). After stirring at same temperature for 2 hour and at room temperature for 1 hours, the solvent was removed under reduced pressure, to give tert-Butyl 4-[2-(methanesulfonyloxymethyl)pyrimidin-5-yl]-3-methyl-3,6-dihydro-2H-pyridine-1-carboxylate.

This crude material, 4-(1,2,4-triazol-1-yl)phenol (12 mg, 77.2 µmol) and potassium iodide (14 mg, 84.9 µmol) was dissolved in tetrahydrofuran (1 mL), added sodium hydride (3.4 mg, 84.9 µmol), and stirred at 70°C for 5 hours. The reaction mixture was cooled to room temperature, poured into water, and was extracted with ethyl acetate. The organic layer was washed with brine, dried over anhydrous sodium sulfate, and the solvent was removed under reduced pressure. The residue was purified by silica gel column chromatography (hexane/ethyl acetate = 1/2), to give the title compound as a yellow amorphous (13 mg, yield 37%).
FAB-MS (m/z) : 449 (M+1)
¹H NMR (CDCl₃, 400 MHz): δ= 1.05 (3H, d, J = 7 Hz), 1.50 (9H, s), 2.7-2.9 (1H, m), 3.33 (1H, dd, J = 4 Hz, 13 Hz), 3.8-4.0 (1H, m), 3.85 (1H, dd, J = 3 Hz,13 Hz), 4.1-4.5 (1H, m), 5.36 (2H, s), 6.02 (1H, br s), 7.14 (2H, d, J = 9 Hz), 7.57 (2H, d, J = 9 Hz), 8.07 (1H, s), 8.44 (1H, s), 8.74 (2H, s).

### [Example 26]

### 5-Ethyl-2-[4-[5-(2,3-difluoro-4-methanesulfonylphenoxymethyl)pyrazin-2-yl]-3,6-dihydro-2H-pyridin-1-yllpyrimidine

To a solution of tert-butyl 4-[5-(2,3-difluoro-4-methanesulfonylphenoxymethyl)pyrazin-2-yl]-3,6-dihydro-2H-pyridine-1-carboxylate (Example 18) (25 mg, 51.9 µmol) in dichloromethane (1 mL) was added trifluoroacetic acid (0.3 mL). After stirring at room temperature for 3 hours, the solvent was removed under reduced pressure. The resulting crude material was dissolved in dry acetonitrile (2 mL), and was added potassium carbonate (36 mg, 0.260 mmol) and 2-chloro-5-ethylpyrimidine (12.4 µL, 0.104 mmol). After stirring at 80°C overnight, cooled to room temperature, the reaction mixture was poured into water, and was extracted with ethyl acetate. The organic layer was washed with brine, dried over anhydrous sodium sulfate, and the solvent was removed under reduced pressure. The residue was purified by silica gel column chromatography (hexane/ethyl acetate = 1/2), to give the title compound as a pale yellow crystal (8.2 mg, yield 32%).
FAB-MS(m/z): 488 (M+1)
¹H NMR (CDCl₃, 400 MHz): δ= 1.20 (3H, t, J = 8 Hz), 2.49 (2H, q, J = 8 Hz), 2.7-2.8 (2H, m), 3.20 (3H, s), 4.0-4.2 (2H, m), 4.4-4.5 (2H, m), 5.38 (2H, s), 6.8-7.0 (1H, m), 6.9-7.1 (1H, m), 7.6-7.8 (1H, m), 8.22 (2H, s), 8.73 (2H, s).
IR (KBr, cm⁻¹): 3010, 2960, 2929, 1604, 1539, 1512, 1489, 1464, 1396, 1365, 1309, 1238, 1169, 1130, 1097, 993, 970, 933, 897, 812, 771, 652, 615, 526, 490, 409.

### [Example 27]

### tert-Butyl 4-[5-(7-fluoro-5-methanesulfonylindol-1-ylmethyl)pyrazin-2-yl]-3,6-dihydro-2H-pyridine-1-carboxylate

### (1) 2-Chloro-5-(7-fluoro-5-methanesulfonylindol-1-ylmethyl)pyrazine

The title compound was prepared from 2-chloro-5-chloromethylpyrazine (34 mg, 0.206 mmol) and 7-fluoro-5-methanesulfonylindole (40 mg, 0.188 mmol) by the similar manner as described in Example 4(2) as a brown oil (50 mg, yield 78%).
¹H NMR (CDCl₃, 400 MHz): δ= 3.07 (3H, s), 5.64 (2H, s), 6.7-6.8 (1H, m), 7.37 (1H, d, J = 3 Hz), 7.42 (1H, d, J = 11 Hz), 8.07 (1H, s), 8.16 (1H, s), 8.52 (1H, s).

### (2) tert-Butyl 4-[5-(7-fluoro-5-methanesulfonylindol-1-ylmethyl)pyrazin-2-yl]-3,6-dihydro-2H-pyridine-1-carboxylate

The title compound was prepared from 2-chloro-5-(7-fluoro-5-methanesulfonylindol-1-ylmethyl)pyrazine (50 mg, 0.142 mmol) and tert-butyl 4-(4,4,5,5-tetramethyl-1,3,2-dioxaborolan-2-yl)-3,6-dihydro-2H-pyridine-1-carboxylate (50 mg, 0.162 mmol) by the similar manner as described in Example 1(1) as a colorless oil (59 mg, yield 82%).
¹H NMR (CDCl₃, 400 MHz): δ= 1.48 (9H, s), 2.5-2.7 (2H, m), 3.06 (3H, s), 3.6-3.7 (2H, m), 4.1-4.2 (2H, m), 5.64 (2H, s), 6.65 (1H, br s), 6.7-6.8 (1H, m), 7.39 (1H, d, J = 3 Hz), 7.41 (1H, dd, J = 1Hz, 10 Hz), 8.06 (1H, d, J = 1 Hz), 8.29 (1H, s), 8.62 (1H, d, J = 1 Hz).

### [Example 28]

### tert-Butyl 4-[5-(7-fluoro-5-methanesulfonylindol-1-ylmethyl)pyrazin-2-yl]piperidine-1-carboxylate

The title compound was prepared from tert-butyl 4-[5-(7-fluoro-5-methanesulfonylindol-1-ylmethyl)pyrazin-2-yl]-3,6-dihydro-2H-pyridine-1-carboxylate (Example 27) (59 mg, 0.121 mmol) by the similar manner as described in Example 1(2) as a white amorphous (43 mg, yield 73%).
FAB-MS(m/z): 488 (M)
¹H NMR (CDCl₃, 400 MHz): δ= 1.46 (9H, s), 1.6-1.8 (2H, m), 1.8-1.9 (2H, m), 2.7-3.0 (3H, m), 3.06 (3H, s), 4.1-4.3 (2H, m), 5.62 (2H, s), 6.75 (1H, t, J = 3 Hz), 7.39 (1H, d, J = 3 Hz), 7.42 (1H, dd, J = 1 Hz, 10 Hz), 8.06 (1H, d, J = 1 Hz), 8.29 (1H, s), 8.40 (1H, d, J = 1 Hz).

### [Example 29]

### Isopropyl 4-[5-(6,7-difluoro-5-methanesulfonylindol-1-ylmethyl)pyrazin-2-yl]-3-methyl-3,6-dihydro-2H-pyridine-1-carboxylate

The title compound was prepared from 2-chloro-5-(6,7-difluoro-5-methanesulfonylindol-1-ylmethyl)pyrazine (Example 20(1)) (37 mg, 0.103 mmol) and isopropyl 3-methyl-4-(4,4,5,5-tetramethyl-1,3,2-dioxaborolan-2-yl)-3,6-dihydro-2H-pyridine-1-carboxylate (32 mg, 0.103 mmol) by the similar manner as described in Example 1(1) as a colorless oil (5.2 mg, yield 10%).
FAB-MS (m/z) : 505 (M+1)
¹H NMR (CDCl₃, 400 MHz): δ= 1.07 (3H, d, J = 6 Hz), 1.2-1.3 (6H, m), 3.1-3.2 (2H, m), 3.23 (3H, s), 3.7-4.1 (2H, m), 4.3-4.6 (1H, m), 4.9-5.1 (1H, m), 5.60 (2H, s), 6.50 (1H, br s), 6.71 (1H, t, J = 3 Hz), 7.35 (1H, d, J = 3 Hz), 8.00 (1H, d, J = 6 Hz), 8.32 (1H, s), 8.64 (1H, s).

### [Example 30]

### Isopropyl 4-[5-(4,6-difluoro-5-methanesulfonylindol-1-ylmethyl)pyrazin-2-yl]-3-methyl-3,6-dihydro-2H-pyridine-1-carboxylate

The title compound was prepared from 2-chloro-5-(4,6-difluoro-5-methanesulfonylindol-1-ylmethyl)pyrazine (Example 16(1)) (27 mg, 0.075 mmol) and isopropyl 3-methyl-4-(4,4,5,5-tetramethyl-1,3,2-dioxaborolan-2-yl)-3,6-dihydro-2H-pyridine-1-carboxylate (23 mg, 0.075 mmol) by the similar manner as described in Example 1(1) as a colorless oil (8.9 mg, yield 23%).
FAB-MS (m/z) : 505 (M+1)
¹H NMR (CDCl₃, 400 MHz): δ= 1.07 (3H, d, J = 6 Hz), 1.2-1.3 (6H, m), 3.0-3.3 (2H, m), 3.30 (3H, s), 3.7-4.2 (2H, m), 4.3-4.7 (1H, m), 4.9-5.1 (1H, m), 5.40 (2H, s), 6.52 (1H, br s), 6.77 (1H, d, J = 3 Hz), 7.03 (1H, d, J = 10 Hz), 7.28 (1H, d, J = 3 Hz), 8.27 (1H, s), 8.66 (1H, s).

### [Example 31]

### tert-Butyl 4-[5-(7-fluoro-5-nitroindol-1-ylmethyl)pyrazin-2-yl]-3,6-dihydro-2H-pyridine-1-carboxylate

### (1) 2-Chloro-5-(7-fluoro-5-nitroindol-1-ylmethyl)pyrazine

The title compound was prepared from 2-chloro-5-chloromethylpyrazine (109 mg, 0.669 mmol) and 7-fluoro-5-nitroindole (120 mg, 0.669 mmol) by the similar manner as described in Example 4(2) as pale yellow crystal (165 mg, yield 68%).
¹H NMR (CDCl₃, 400 MHz): δ= 5.63 (2H, s), 6.80 (1H, t, J = 3 Hz), 7.37 (1H, d, J = 3 Hz), 7.81 (1H, dd, J = 2 Hz, 12 Hz), 8.19 (1H, s), 8.41 (1H, d, J = 2 Hz), 8.53 (1H, d, J = 1 Hz).

### (2) tert-Butyl 4-[5-(7-fluoro-5-nitroindol-1-ylmethyl)pyrazin-2-yl]-3,6-dihydro-2H-pyridine-1-carboxylate

The title compound was prepared from 2-chloro-5-(7-fluoro-5-nitroindol-1-ylmethyl)pyrazine (475 mg, 1.55 mmol) and tert-butyl 4-(4,4,5,5-tetramethyl-1,3,2-dioxaborolan-2-yl)-3,6-dihydro-2H-pyridine-1-carboxylate (575 mg, 1.86 mmol) by the similar manner as described in Example 1(1) as a pale yellow crystal (431 mg, yield 61%).
¹H NMR (CDCl₃, 400 MHz): δ= 1.48 (9H, s), 2.5-2.7 (2H, m), 3.5-3.7 (2H, m), 4.1-4.2 (2H, m), 5.63 (2H, s), 6.66 (1H, br s), 6.79 (1H, t, J = 3 Hz), 7.39 (1H, d=3 Hz), 7.80 (1H, dd, J = 2 Hz, 12 Hz), 8.32 (1H, s), 8.40 (1H, d, J = 2 Hz), 8.63 (1H, d, J = 1 Hz).

### [Example 32]

### tert-Butyl 4-[5-(5-amino-7-fluoroindol-1-ylmethyl)pyrazin-2-yl]-3,6-dihydro-2H-pyridine-1-carboxylate

The title compound was prepared from tert-butyl 4-[5-(7-fluoro-5-nitroindol-1-ylmethyl)pyrazin-2-yl]-3,6-dihydro-2H-pyridine-1-carboxylate (Example 31) (66 mg, 0.146 mmol) by the similar manner as described in Example 6 as a brown oil (38 mg, yield 61%).
¹H NMR (CDCl₃, 400 MHz): δ= 1.48 (9H, s), 2.5-2.7 (2H, m), 3.52 (2H, br s), 3.5-3.7 (2H, m), 4.1-4.2 (2H, m), 5.50 (2H, s), 6.3-6.4 (2H, m), 6.62 (1H, br s), 6.66 (1H, d, J = 2 Hz), 7.10 (1H, d, J = 3 Hz), 8.14 (1H, s), 8.61 (1H, d, J = 1 Hz).

### [Example 33]

### tert-Butyl 4-[5-[7-fluoro-5-(tetrazol-1-yl)indol-1-ylmethyl]pyrazin-2-yl]-3,6-dihydro-2H-pyridine-1-carboxylate

The title compound was prepared from tert-butyl 4-[5-(5-amino-7-fluoroindol-1-ylmethyl)pyrazin-2-yl]-3,6-dihydro-2H-pyridine-1-carboxylate (Example 32) (38 mg, 89.7 µmol) by the similar manner as described in Example 7 as a brown oil (35 mg, yield 81%).
¹H NMR (CDCl₃, 400 MHz): δ= 1.48 (9H, s), 2.5-2.7 (2H, m), 3.5-3.7 (2H, m), 4.1-4.2 (2H, m), 5.64 (2H, s), 6.66 (1H, br s), 6.71 (1H, t, J = 3 Hz), 7.2-7.3 (1H, m), 7.39 (1H, d, J = 3 Hz), 7.69 (1H, d, J = 1 Hz), 8.30 (1H, s), 8.64 (1H, s), 8.94 (1H, s).

### [Example 34]

### tert-Butyl 4-[5-[7-fluoro-5-(tetrazol-1-yl)indol-1-ylmethyl]pyrazin-2-yl]piperidine-1-carboxylate

The title compound was prepared from tert-butyl 4-[5-[7-fluoro-5-(tetrazol-1-yl)indol-1-ylmethyl]pyrazin-2-yl]-3,6-dihydro-2H-pyridine-1-carboxylate (Example 33) (34 mg, 71.4 µmol) by the similar manner as described in Example 1(2) as a pale brown amorphous (24 mg, yield 70%).
FAB-MS (m/z) : 479 (M+1)
¹H NMR (CDCl₃, 400 MHz): δ= 1.46 (9H, s), 1.6-1.8 (2H, m), 1.8-1.9 (2H, m), 2.7-3.0 (3H, m), 4.1-4.4 (2H, m), 5.63 (2H, s), 6.71 (1H, t, J = 3 Hz), 7.26 (1H, dd, J = 2Hz,12 Hz), 7.39 (1H, d, J = 3 Hz), 7.69 (1H, d, J = 2 Hz), 8.30 (1H, s), 8.41 (1H, d, J = 1 Hz), 8.94 (1H, s).

### [Example 35]

### Isopropyl 4-[5-(7-fluoro-5-nitroindol-1-ylmethyl)pyrazin-2-yl]-3,6-dihydro-2H-pyridine-1-carboxylate

A solution of tert-butyl 4-[5-(7-fluoro-5-nitroindol-1-ylmethyl)pyrazin-2-yl]-3,6-dihydro-2H-pyridine-1-carboxylate (Example 31) (80 mg, 0.176 mmol) in trifluoroacetic acid (0.8 mL) was stirred at room temperature for 1 hour, and then the solvent was removed under reduced pressure. The resulting mixture was dissolved in tetrahydrofuran (1 mL)-water (1 mL), and was added triethylamine (0.25 mL, 1.76 mmol) and isopropyl chloroformate (30 µL, 0.265 mmol) under ice-cooling. After stirring at room temperature for 2 hours, the reaction mixture was added water, and was extracted with ethyl acetate. The organic layer was washed with brine, dried over anhydrous sodium sulfate, and the solvent was removed under reduced pressure. The residue was purified by silica gel column chromatography (hexane/ethyl acetate = 2/1 → 3/2), to give the title compound as a pale yellow crystal (76 mg, yield 98%).
¹H NMR (CDCl₃, 400 MHz): δ= 1.26 (6H, d, J = 6 Hz), 2.5-2.7 (2H, m), 3.6-3.8 (2H, m), 4.1-4.2 (2H, m), 4.8-5.0 (1H, m), 5.63 (2H, s), 6.66 (1H, br s), 6.79 (1H, t, J = 3 Hz), 7.39 (1H, d=3 Hz), 7.80 (1H, dd, J = 2 H, 12 Hz), 8.32 (1H, s), 8.40 (1H, d, J = 2 Hz), 8.63 (1H, d, J = 1 Hz).

### [Example 36]

### Isopropyl 4-[5-(5-amino-7-fluoroindol-1-ylmethyl)pyrazin-2-yl]-3,6-dihydro-2H-pyridine-1-carboxylate

The title compound was prepared from isopropyl 4-[5-(7-fluoro-5-nitroindol-1-ylmethyl)pyrazin-2-yl]-3,6-dihydro-2H-pyridine-1-carboxylate (Example 35) (75 mg, 0.171 mmol) by the similar manner as described in Example 6 as a brown oil (42 mg, yield 60%).
¹H NMR (CDCl₃, 400 MHz): δ= 1.26 (6H, d, J = 6 Hz), 2.5-2.7 (2H, m), 3.52 (2H, br s), 3.5-3.7 (2H, m), 4.1-4.2 (2H, m), 4.9-5.0 (1H, m), 5.50 (2H, s), 6.3-6.4 (2H, m), 6.62 (1H, br s), 6.65 (1H, d, J = 2 Hz), 7.10 (1H, d, J = 3 Hz), 8.14 (1H, s), 8.61 (1H, d, J = 1 Hz).

### [Example 37]

### Isopropyl 4-[5-[7-fluoro-5-(tetrazol-1-yl)indol-1-ylmethyl]pyrazin-2-yl]-3,6-dihydro-2H-pyridine-1-carboxylate

The title compound was prepared from isopropyl 4-[5-(5-amino-7-fluoroindol-1-ylmethyl)pyrazin-2-yl]-3,6-dihydro-2H-pyridine-1-carboxylate (Example 36) (42 mg, 0.103 mmol) by the similar manner as described in Example 7 as a brown oil (36 mg, yield 76%).
¹H NMR (CDCl₃, 400 MHz): δ= 1.26 (6H, d, J = 6 Hz), 2.5-2.7 (2H, m), 3.5-3.7 (2H, m), 4.1-4.2 (2H, m), 4.8-5.0 (1H, m), 5.65 (2H, s), 6.6-6.8 (2H, m), 7.2-7.3 (1H, m), 7.39 (1H, d, J = 3 Hz), 7.69 (1H, s), 8.30 (1H, s), 8.64 (1H, s), 8.94 (1H, s).

### [Example 38]

### Isopropyl 4-[5-[7-fluoro-5-(tetrazol-1-yl)indol-1-ylmethyl]pyrazin-2-yl]piperidine-1-carboxylate

The title compound was prepared from isopropyl 4-[5-[7-fluoro-5-(tetrazol-1-yl)indol-1-ylmethyl]pyrazin-2-yl]-3,6-dihydro-2H-pyridine-1-carboxylate (Example 37) (36 mg, 77.8 µmol) by the similar manner as described in Example 1(2) as a pale brown amorphous (28 mg, yield 78%).
FAB-MS(m/z): 465 (M+1)
¹H NMR (CDCl₃, 400 MHz): δ= 1.24 (6H, d, J = 6 Hz), 1.6-1.8 (2H, m), 1.8-2.0 (2H, m), 2.7-3.0 (3H, m), 4.1-4.4 (2H, m), 4.8-5.0 (1H, m), 5.63 (2H, s), 6.6-6.8 (1H, m), 7.2-7.3 (1H, m), 7.39 (1H, d, J = 3 Hz), 7.69 (1H, d, J = 1 Hz), 8.30 (1H, s), 8.41 (1H, s), 8.94 (1H, s).

### [Example 39]

### 5-Ethyl-2-[4-[5-(7-fluoro-5-nitroindol-1-ylmethyl)pyrazin-2-yl]-3,6-dihydro-2H-pyridin-1-yl]pyrimidine

The title compound was prepared from tert-butyl 4-[5-(7-fluoro-5-nitroindol-1-ylmethyl)pyrazin-2-yl]-3,6-dihydro-2H-pyridine-1-carboxylate (Example 31) (120 mg, 0.265 mmol) by the similar manner as described in Example 26 as a pale yellow crystal (68 mg, yield 56%).
¹H NMR (CDCl₃, 400 MHz): δ= 1.19 (3H, t, J = 8 Hz), 2.48 (2H, q, J = 8 Hz), 2.6-2.8 (2H, m), 4.0-4.2 (2H, m), 4.4-4.5 (2H, m), 5.63 (2H, s), 6.7-6.9 (2H, m), 7.39 (1H, d=3 Hz), 7.80 (1H, dd, J = 2 Hz, 12 Hz), 8.21 (2H, s), 8.33 (1H, s), 8.40 (1H, d, J = 2 Hz), 8.66 (1H, d, J = 1 Hz).

### [Example 40]

### 2-[4-[5-(5-Amino-7-fluoroindol-1-ylmethyl)pyrazin-2-yl]-3,6-dihydro-2H-pyridin-1-yl]-5-ethylpyrimidine

The title compound was prepared from 5-ethyl-2-[4-[5-(7-fluoro-5-nitroindol-1-ylmethyl)pyrazin-2-yl]-3,6-dihydro-2H-pyridin-1-yl]pyrimidine (Example 39) (67 mg, 0.146 mmol) by the similar manner as described in Example 6 as a brown oil (17 mg, yield 27%).
¹H NMR (CDCl₃, 400 MHz): δ= 1.19 (3H, t, J = 7 Hz), 2.47 (2H, q, J = 7 Hz), 2.6-2.8 (2H, m), 3.52 (2H, br s), 4.0-4.1 (2H, m), 4.4-4.5 (2H, m), 5.50 (2H, s), 6.3-6.4 (2H, m), 6.65 (1H, d, J = 2 Hz), 6.7-6.8 (1H, m), 7.10 (1H, d, J = 3 Hz), 8.14 (1H, s), 8.21 (2H, s), 8.65 (1H, d, J = 1 Hz).

### [Example 41]

### 5-Ethyl-2-[4-[5-[7-fluoro-5-(tetrazol-1-yl)indol-1-ylmethyl]pyrazin-2-yl]-3,6-dihydro-2H-pyridin-1-yl]pyrimidine

The title compound was prepared from 2-[4-[5-(5-amino-7-fluoroindol-1-ylmethyl)pyrazin-2-yl]-3,6-dihydro-2H-pyridin-1-yl]-5-ethylpyrimidine (Example 40) (17 mg, 39.6 µmol) by the similar manner as described in Example 7 as a brown oil (14 mg, yield 73%).
¹H NMR (CDCl₃, 400 MHz): δ= 1.19 (3H, t, J = 7 Hz), 2.47 (2H, q, J = 7 Hz), 2.6-2.8 (2H, m), 4.0-4.1 (2H, m), 4.4-4.5 (2H, m), 5.64 (2H, s), 6.6-6.9 (2H, m), 7.2-7.3 (1H, m), 7.39 (1H, d, J = 3 Hz), 7.69 (1H, d, J = 1 Hz), 8.21 (2H, s), 8.30 (1H, s), 8.67 (1H, s), 8.94 (1H, s).

### [Example 42]

### 5-Ethyl-2-[4-[5-[7-fluoro-5-(tetrazol-1-yl)indol-1-ylmethyl]pyrazin-2-yl]piperidin-1-yl]pyrimidine

The title compound was prepared from 5-ethyl-2-[4-[5-[7-fluoro-5-(tetrazol-1-yl)indol-1-ylmethyl]pyrazin-2-yl]-3,6-dihydro-2H-pyridin-1-yl]pyrimidine (Example 41) (14 mg, 77.8 µmol) by the similar manner as described in Example 1(2) as a pale brown crystal (12 mg, yield 92%).
FAB-MS(m/z): 485 (M+1)
¹H NMR (CDCl₃, 400 MHz): δ= 1.19 (3H, t, J = 7 Hz), 1.7-1.9 (2H, m), 1.9-2.0 (2H, m), 2.46 (2H, q, J = 7 Hz), 2.9-3.1 (3H, m), 4.7-5.0 (2H, m), 5.63 (2H, s), 6.6-6.8 (1H, m), 7.2-7.3 (1H, m), 7.39 (1H, d, J = 3 Hz), 7.69 (1H, d, J = 1 Hz), 8.17 (2H, s), 8.30 (1H, s), 8.43 (1H, s), 8.93 (1H, s).

### [Example 43]

### tert-Butyl 4-[5-(5-methanesulfonyl-1H-pyrrolo[2,3-b]pyridin-1-ylmethyl)pyrazin-2-yl]piperidine-1-carboxylate

The title compound was prepared from tert-butyl 4-[5-(5-methanesulfonyl-1H-pyrrolo[2,3-b]pyridin-1-ylmethyl)pyrazin-2-yl]-3,6-dihydro-2H-pyridine-1-carboxylate (Example 14) (53 mg, 0.113 mmol) by the similar manner as described in Example 1(2) as a white amorphous (48 mg, yield 90%).
FAB-MS(m/z): 471 (M)
¹H NMR (CDCl₃, 400 MHz): δ= 1.46 (9H, s), 1.6-1.8 (2H, m), 1.8-1.9 (2H, m), 2.7-2.9 (3H, m), 3.12 (3H, s), 4.2-4.4 (2H, m), 5.66 (2H, s), 6.69 (1H, d, J = 3 Hz), 7.55 (1H, d, J = 3 Hz), 8.40 (1H, d, J = 1 Hz), 8.45 (1H, s), 8.49 (1H, d, J = 1 Hz), 8.87 (1H, d, J = 1 Hz).

### [Example 44]

### Isopropyl 4-[5-(5-methanesulfonyl-1H-pyrrolo[2,3-b]pyridin-1-ylmethyl)pyrazin-2-yl]piperidine-1-carboxylate

The title compound was prepared from tert-butyl 4-[5-(5-methanesulfonyl-1H-pyrrolo[2,3-b]pyridin-1-ylmethyl)pyrazin-2-yl]piperidine-1-carboxylate (Example 43) (20 mg, 0.0424 mmol) and 5-bromo-2-chloropyrimidine (16 mg, 0.0848 mmol) by the similar manner as described in Example 35 as a white amorphous (18 mg, yield 93%).
FAB-MS(m/z): 458 (M+1)
¹H NMR (CDCl₃, 400 MHz): δ= 1.24 (6H, d, J = 6 Hz), 1.6-1.8 (2H, m), 1.8-1.9 (2H, m), 2.8-3.0 (3H, m), 3.12 (3H, s), 4.2-4.4 (2H, m), 4.8-5.0 (1H, m), 5.66 (2H, s), 6.69 (1H, d, J = 3 Hz), 7.54 (1H, d, J = 3 Hz), 8.40 (1H, d, J = 1 Hz), 8.45 (1H, s), 8.49 (1H, d, J = 2 Hz), 8.87 (1H, d, J = 2 Hz).

### [Example 45]

### 5-Bromo-2-[4-[5-(5-methanesulfonyl-1H-pyrrolo[2,3-b]pyridin-1-ylmethyl)pyrazin-2-yl]piperidine-1-yl]pyrimidine

The title compound was prepared from tert-butyl 4-[5-(5-methanesulfonyl-1H-pyrrolo[2,3-b]pyridin-1-ylmethyl)pyrazin-2-yl]piperidine-1-carboxylate (Example 43) (20 mg, 0.0424 mmol) by the similar manner as described in Example 26 as a pale yellow crystal (13 mg, yield 58%).
FAB-MS(m/z): 528 (M+1)
¹H NMR (CDCl₃, 400 MHz): δ= 1.7-1.9 (2H, m), 1.9-2.0 (2H, m), 2.9-3.1 (3H, m), 3.12 (3H, s), 4.8-4.9 (2H, m), 5.66 (2H, s), 6.69 (1H, d, J = 3 Hz), 7.54 (1H, d, J = 3 Hz), 8.28 (2H, s), 8.42 (1H, d, J = 1 Hz), 8.44 (1H, d, J = 1 Hz), 8.49 (1H, d, J = 2 Hz), 8.86 (1H, d, J = 2 Hz).

### [Example 46]

### tert-Butyl 4-[2-(5-methanesulfonyl-1H-pyrrolo[2,3-b]pyridin-1-ylmethyl)pyrimidin-5-yl]piperidine-1-carboxylate

### (1) 5-Bromo-2-(tert-butyldimethylsilyloxymethyl)pyrimidine

To a solution of 5-bromo-2-hydroxymethylpyrimidine (177 mg, 0.937 mmol) in N,N-dimethylformamide (9.4 mL) was added triethylamine (0.26 mL, 1.87 mmol) and tertbutyldimethylsilyl chloride (155 mg, 1.03 mmol) under N₂. After stirring at room temperature for 2 hours, the reaction mixture was added water, and was extracted with ethyl acetate. The organic layer was washed with brine, dried over anhydrous sodium sulfate, and the solvent was removed under reduced pressure. The residue was purified by silica gel column chromatography (hexane/ethyl acetate = 100/1 → 4/1), to give the title compound as a pale yellow oil (215 mg, yield 76%).
¹H NMR (CDCl₃, 400 MHz): δ= 0.13 (6H, s), 0.94 (9H, s), 4.88 (2H, s), 8.77 (2H, s).

### (2) tert-Butyl 4-[2-(tert-butyldimethylsilyloxymethyl)pyrimidin-5-yl]-3,6-dihydro-2H-pyridine-1-carboxylate

The title compound was prepared from 5-bromo-2-(tert-butyldimethylsilyloxymethyl)pyrimidine (215 mg, 0.709 mmol) and tert-butyl 4-(4,4,5,5-tetramethyl-1,3,2-dioxaborolan-2-yl)-3,6-dihydro-2H-pyridine-1-carboxylate (219 mg, 0.709 mmol) by the similar manner as described in Example 1(1) as a pale yellow crystal (293 mg, yield 100%).
¹H NMR (CDCl₃, 400 MHz): δ= 0.14 (6H, s), 0.94 (9H, s), 1.49 (9H, s), 2.4-2.6 (2H, m), 3.6-3.7 (2H, m), 4.0-4.2 (2H, m), 4.92 (2H, s), 6.15 (1H, br s), 8.72 (2H, s).

### (3) tert-Butyl 4-(2-hydroxymethylpyrimidin-5-yl)-3,6-dihydro-2H-pyridine-1-carboxylate

tert-Butyl 4-[2-(tert-butyldimethylsilyloxymethyl)pyrimidin-5-yl]-3,6-dihydro-2H-pyridine-1-carboxylate (435 mg, 1.07 mmol) was dissolved in dry tetrahydrofuran, and was added dropwise 1.0 M tetrabutylammonium fluoride-tetrahydrofuran solution (1.3 mL, 1.29 mmol). After stirring at room temperature for 3 hours, the reaction mixture was added added water, and extracted wit ethyl acetate. The organic layer was washed with brine, dried over anhydrous sodium sulfate, and the solvent was removed under reduced pressure. The residue was purified by silica gel column chromatography (hexane/ethyl acetate = 6/1 → 0/100), to give the title compound as a pale yellow oil(296 mg, yield 86%).
¹H NMR (CDCl₃, 400 MHz): δ= 1.50 (9H, s), 2.5-2.6 (2H, m), 3.59 (1H, t, J = 5 Hz), 3.6-3.7 (2H, m), 4.1-4.2 (2H, m), 4.84 (2H, d, J = 5 Hz), 6.17 (1H, br s), 8.72 (2H, s).

### (4) tert-Butyl 4-(2-hydroxymethylpyrimidin-5-yl)piperidine-1-carboxylate

The title compound was prepared from tert-butyl 4-(2-hydroxymethylpyrimidin-5-yl)-3,6-dihydro-2H-pyridine-1-carboxylate (296 mg, 1.02 mmol) by the similar manner as described in Example 1(2) as a colorless oil (133 mg, yield 47%).
¹H NMR (CDCl₃, 400 MHz): δ= 1.49 (9H, s), 1.5-1.8 (2H, m), 1.8-1.9 (2H, m), 2.6-2.8 (1H, m), 2.8-2.9 (2H, m), 3.58 (1H, br s), 4.2-4.4 (2H, m), 4.82 (2H, s), 8.59 (2H, s).

### (5) tert-Butyl 4-[2-(5-methanesulfonyl-1H-pyrrolo[2,3-b]pyridin-1-ylmethyl)pyrimidin-5-yl]piperidine-1-carboxylate

The title compound was prepared from tert-butyl 4-(2-hydroxymethylpyrimidin-5-yl)piperidine-1-carboxylate (40 mg, 0.136 mmol) by the similar manner as described in Example 4(1) and 4(2) as a yellow amorphous (35 mg, yield 69%).
FAB-MS(m/z): 472 (M+1)
¹H NMR (CDCl₃, 400 MHz): δ= 1.47 (9H, s), 1.5-1.7 (2H, m), 1.7-1.9 (2H, m), 2.6-2.7 (1H, m), 2.7-2.9 (2H, m), 3.11 (3H, s), 4.2-4.4 (2H, m), 5.78 (2H, s), 6.71 (1H, d, J = 3 Hz), 7.54 (1H, d, J = 3 Hz), 8.49 (1H, d, J = 2 Hz), 8.52 (2H, s), 8.85 (1H, d, J = 2 Hz).

### [Example 47]

### tert-Butyl 4-[2-(7-fluoro-5-nitroindol-1-ylmethyl)pyrimidin-5-yl]piperidine-1-carboxylate

The title compound was prepared from tert-butyl 4-(2-hydroxymethylpyrimidin-5-yl)piperidine-1-carboxylate (Example 46(4)) (93 mg, 0.317 mmol) and 7-fluoro-5-nitroindole (63 mg, 0.351 mmol) by the similar manner as described in Example 4(1) and 4(2) as a yellow crystal (111 mg, yield 70%).
¹H NMR (CDCl₃, 400 MHz): δ= 1.47 (9H, s), 1.5-1.7 (2H, m), 1.7-1.9 (2H, m), 2.6-2.7 (1H, m), 2.7-2.9 (2H, m), 4.2-4.4 (2H, m), 5.71 (2H, s), 6.79 (1H, t, J = 3 Hz), 7.33 (1H, d, J = 3 Hz), 7.77 (1H, dd, J = 2 Hz, 10 Hz), 8.42 (1H, d, J = 2 Hz), 8.51 (2H, s).

### [Example 48]

### tert-Butyl 4-[2-(5-amino-7-fluoroindol-1-ylmethyl)pyrimidin-5-yl]piperidine-1-carboxylate

The title compound was prepared from tert-butyl 4-[2-(7-fluoro-5-nitroindol-1-ylmethyl)pyrimidin-5-yl]piperidine-1-carboxylate (Example 47) (111 mg, 0.244 mmol) by the similar manner as described in Example 6 as a brown amorphous (97 mg, yield 94%).
¹H NMR (CDCl₃, 400 MHz): δ= 1.47 (9H, s), 1.5-1.7 (2H, m), 1.7-1.9 (2H, m), 2.5-2.7 (1H, m), 2.7-2.9 (2H, m), 4.2-4.4 (2H, m), 5.59 (2H, s), 6.2-6.4 (2H, m), 6.66 (1H, s), 7.10 (1H, d, J = 3 Hz), 8.51 (2H, s).

### [Example 49]

### tert-Butyl 4-[2-[7-fluoro-5-(tetrazol-1-yl)indol-1-ylmethyl]pyrimidin-5-yl]piperidine-1-carboxylate

The title compound was prepared from tert-butyl 4-[2-(5-amino-7-fluoroindol-1-ylmethyl)pyrimidin-5-yl]piperidine-1-carboxylate (Example 48) (97 mg, 0.228 mmol) by the similar manner as described in Example 7 as a brown amorphous (57 mg, yield 52%).
FAB-MS(m/z): 479 (M+1)
¹H NMR (CDCl₃, 400 MHz): δ= 1.47 (9H, s), 1.5-1.7 (2H, m), 1.7-1.9 (2H, m), 2.6-2.9 (3H, m), 4.2-4.3 (2H, m), 5.73 (2H, s), 6.72 (1H, t, J = 3 Hz), 7.22 (1H, dd, J = 2 Hz,12 Hz), 7.35 (1H, d, J = 3 Hz), 7.69 (1H, d, J = 2 Hz), 8.53 (2H, s), 8.94 (1H, s).

### [Example 50]

### Isopropyl 4-[2-[7-fluoro-5-(tetrazol-1-yl)indol-1-ylmethyl]pyrimidin-5-yl]piperidine-1-carboxylate

The title compound was prepared from tert-butyl 4-[2-[7-fluoro-5-(tetrazol-1-yl)indol-1-ylmethyl]pyrimidin-5-yl]piperidine-1-carboxylate (Example 49) (45 mg, 0.0940 mmol) by the similar manner as described in Example 35 as a brown amorphous (34 mg, yield 78%).
FAB-MS(m/z): 465 (M+1)
¹H NMR (CDCl₃, 400 MHz): δ= 1.25 (6H, d, J = 6 Hz), 1.5-1.7 (2H, m), 1.8-1.9 (2H, m), 2.6-2.9 (3H, m), 4.2-4.4 (2H, m), 4.9-5.0 (1H, m), 5.73 (2H, s), 6.72 (1H, t, J = 3 Hz), 7.22 (1H, dd, J = 2 Hz, 12 Hz), 7.34 (1H, d, J = 3 Hz), 7.69 (1H, d, J = 2 Hz), 8.53 (2H, s), 8.93 (1H, s).

### [Example 51]

### 5-Bromo-2-[4-[5-(7-fluoro-5-methanesulfonylindol-1-ylmethyl)pyrazin-2-yl]piperidine-1-yl]pyrimidine

The title compound was prepared from tert-butyl 4-[5-(7-fluoro-5-methanesulfonylindol-1-ylmethyl)pyrazin-2-yl]piperidine-1-carboxylate (Example 28) (33 mg, 0.0675 mmol) by the similar manner as described in Example 26 as a pale yellow crystal (26 mg, yield 71%).
FAB-MS(m/z): 545 (M+1)
¹H NMR (CDCl₃, 400 MHz): δ= 1.7-1.9 (2H, m), 1.9-2.0 (2H, m), 2.9-3.1 (3H, m), 3.06 (3H, s), 4.8-4.9 (2H, m), 5.62 (2H, s), 6.7-6.8 (1H, m), 7.38 (1H, d, J = 3 Hz), 7.42 (1H, dd, J = 1 Hz,10 Hz), 8.06 (1H, d, J = 1 Hz), 8.28 (2H, s), 8.29 (1H, s), 8.42 (1H, d, J = 1 Hz).

### [Example 52]

### tert-Butyl 4-[5-(5-amino-7-fluoroindol-1-ylmethyl)pyrazin-2-yl]piperidine-1-carboxylate

To a solution of tert-Butyl 4-[5-(7-fluoro-5-nitroindol-1-ylmethyl)pyrazin-2-yl]-3,6-dihydro-2H-pyridine-1-carboxylate (Example 31) (150 mg, 0.331 mmol) in methanol (1.6 mL)-tetrahydrofuran (1.6 mL) was added 5% palladium-carbon (wetted with ca. 52% Water, BNA-5D(N.E. CHEMCAT Corporation)) (15 mg) and then the mixture was hydrogenated at room temperature for 3 hours under 1 atm of H₂. The reaction mixture was filtered through a celite pad, and the filtrate was concentrated under reduced pressure. The residue was purified by silica gel column chromatography (hexane/ethyl acetate = 1/1), to give the title compound as a yellow amorphous (125 mg, yield 89%).
¹H NMR (CDCl₃, 400 MHz): δ= 1.46 (9H, s), 1.6-1.8 (2H, m), 1.8-1.9 (2H, m), 2.7-2.9 (3H, m), 3.52 (2H, br s), 4.1-4.3 (2H, m), 5.49 (2H, s), 6.34 (1H, dd, J = 2 Hz,8 Hz), 6.37 (1H, d, J = 3 Hz), 6.65 (1H, d, J = 2 Hz), 7.10 (1H, d, J = 3 Hz), 8.16 (1H, s), 8.39 (1H, d, J = 2 Hz).

### [Example 53]

### 2-[4-[5-(5-Amino-7-fluoroindol-1-ylmethyl)pyrazin-2-yl]piperidin-1-yl]-5-bromopyrimidine

The title compound was prepared from tert-butyl 4-[5-(5-amino-7-fluoroindol-1-ylmethyl)pyrazin-2-yl]piperidine-1-carboxylate (Example 52) (25 mg, 0.588 mmol) and 5-bromo-2-chloropyrimidine (16 mg, 0.0811 mmol) by the similar manner as described in Example 26 as a brown oil (19 mg, yield 67%).
¹H NMR (CDCl₃, 400 MHz): δ= 1.7-1.9 (2H, m), 1.9-2.0 (2H, m), 2.9-3.1 (3H, m), 3.50 (2H, br s), 4.8-4.9 (2H, m), 5.48 (2H, s), 6.3-6.4 (1H, m), 6.36 (1H, d, J = 3 Hz), 6.65 (1H, d, J = 1 Hz), 7.10 (1H, d, J = 3 Hz), 8.16 (1H, s), 8.27 (2H, s), 8.40 (1H, s).

### [Example 54]

### 5-Bromo-2-[4-[5-[7-fluoro-5-(tetrazol-1-yl)indol-1-ylmethyl]pyrazin-2-yl]piperidin-1-yl]pyrimidine

The title compound was prepared from 2-[4-[5-(5-amino-7-fluoroindol-1-ylmethyl)pyrazin-2-yl]piperidin-1-yl]-5-bromopyrimidine (Example 53) (19 mg, 0.0394 mmol) by the similar manner as described in Example 7 as a pale yellow amorphous (17 mg, yield 81%).
FAB-MS(m/z): 535 (M+1)
¹H NMR (CDCl₃, 400 MHz): δ= 1.7-1.9 (2H, m), 1.9-2.0 (2H, m), 2.9-3.1 (3H, m), 4.8-4.9 (2H, m), 5.63 (2H, s), 6.7-6.8 (1H, m), 7.26 (1H, dd, J = 2 Hz, 11 Hz), 7.38 (1H, d, J = 3 Hz), 7.69 (1H, d, J = 2 Hz), 8.28 (2H, s), 8.2-8.4 (1H, m), 8.43 (1H, d, J = 2 Hz), 8.93 (1H, s).

### [Example 55]

### 2-[4-[5-(5-Amino-7-fluoroindol-1-ylmethyl)pyrazin-2-yl]piperidin-1-yl]-5-trifluoromethylpyrimidine

The title compound was prepared from tert-butyl 4-[5-(5-amino-7-fluoroindol-1-ylmethyl)pyrazin-2-yl]piperidine-1-carboxylate (Example 52) (41 mg, 0.0964mmol) and 2-chloro-5-trifluoromethylpyrimidine (19 mg, 0.106 mmol) by the similar manner as described in Example 26 as a brown crystal (23 mg, yield 51%).
¹H NMR (CDCl₃, 400 MHz): δ= 1.7-1.9 (2H, m), 1.9-2.1 (2H, m), 3.0-3.1 (3H, m), 3.50 (2H, br s), 4.9-5.1 (2H, m), 5.49 (2H, s), 6.34 (1H, dd, J = 2 Hz,10 Hz), 6.36 (1H, d, J = 3 Hz), 6.65 (1H, d, J = 2 Hz), 7.10 (1H, d, J = 3 Hz), 8.16 (2H, s), 8.41 (1H, d, J = 2 Hz), 8.47 (1H, s).

### [Example 56]

### 2-[4-[5-[7-fluoro-5-(tetrazol-1-yl)indol-1-ylmethyl]pyrazin-2-yl]piperidin-1-yl]-5-trifluoromethylpyrimidine

The title compound was prepared from 2-[4-[5-(5-amino-7-fluoroindol-1-ylmethyl)pyrazin-2-yl]piperidin-1-yl]-5-trifluoromethylpyrimidine (Example 55) (23 mg, 0.0488 mmol) by the similar manner as described in Example 7 as a pale brown amorphous (22 mg, yield 86%).
FAB-MS(m/z): 525 (M+1)
¹H NMR (CDCl₃, 400 MHz): δ= 1.7-1.9 (2H, m), 1.9-2.1 (2H, m), 3.0-3.2 (3H, m), 4.9-5.1 (2H, m), 5.63 (2H, s), 6.7-6.8 (1H, m), 7.26 (1H, dd, J = 2 Hz,11 Hz), 7.39 (1H, d, J = 3 Hz), 7.69 (1H, d, J = 2 Hz), 8.30 (1H, s), 8.44 (1H, d, J = 2 Hz), 8.47 (2H, s), 8.93 (1H, s).

### [Example 57]

### 2-[4-[5-(5-Amino-7-fluoroindol-1-ylmethyl)pyrazin-2-yl]piperidin-1-yl]-5-chloropyrimidine

The title compound was prepared from tert-butyl 4-[5-(5-amino-7-fluoroindol-1-ylmethyl)pyrazin-2-yl]piperidine-1-carboxylate (Example 52) (41 mg, 0.0964mmol) and 2,5-dichloropyrimidine (16 mg, 0.106 mmol) by the similar manner as described in Example 26 as a pale yellow oil (1.9 mg, yield 4.5%).
¹H NMR (CDCl₃, 400 MHz): δ= 1.7-1.9 (2H, m), 1.9-2.0 (2H, m), 2.9-3.1 (3H, m), 3.50 (2H, br s), 4.8-4.9 (2H, m), 5.48 (2H, s), 6.34 (1H, dd, J = 2 Hz,10 Hz), 6.36 (1H, d, J = 2 Hz), 6.65 (1H, d, J = 2 Hz), 7.10 (1H, d, J = 2 Hz), 8.16 (1H, s), 8.21 (2H, s), 8.40 (1H, d, J = 2 Hz).

### [Example 58]

### 2-[4-[5-(5-Amino-7-fluoroindol-1-ylmethyl)pyrazin-2-yl]piperidin-1-yl]-5-fluoropyrimidine

The title compound was prepared from tert-butyl 4-[5-(5-amino-7-fluoroindol-1-ylmethyl)pyrazin-2-yl]piperidine-1-carboxylate (Example 52) (41 mg, 0.0964mmol) and 2-chloro-5-fluoropyrimidine (14 mg, 0.106 mmol) by the similar manner as described in Example 26 as a brown oil (6.2 mg, yield 15%).
¹H NMR (CDCl₃, 400 MHz): δ= 1.7-1.9 (2H, m), 1.9-2.0 (2H, m), 2.9-3.1 (3H, m), 3.40 (2H, br s), 4.7-4.9 (2H, m), 5.48 (2H, s), 6.34 (1H, dd, J = 2 Hz, 10 Hz), 6.36 (1H, d, J = 3 Hz), 6.65 (1H, d, J = 2 Hz), 7.10 (1H, d, J = 3 Hz), 8.1-8.2 (1H, m), 8.18 (2H, s), 8.41 (1H, d, J = 2 Hz).

### [Example 59]

### 2-[4-[5-[7-fluoro-5-(tetrazol-1-yl)indol-1-ylmethyl]pyrazin-2-yl]piperidin-1-yl]-5-fluoropyrimidine

The title compound was prepared from 2-[4-[5-(5-amino-7-fluoroindol-1-ylmethyl)pyrazin-2-yl]piperidin-1-yl]-5-fluoropyrimidine (Example 58) (6 mg, 0.0142 mmol) by the similar manner as described in Example 7 as a white amorphous (6 mg, yield 89%).
FAB-MS(m/z): 475 (M+1)
¹H NMR (CDCl₃, 400 MHz): δ= 1.7-1.9 (2H, m), 1.9-2.0 (2H, m), 2.9-3.1 (3H, m), 4.7-4.9 (2H, m), 5.63 (2H, s), 6.6-6.8 (1H, m), 7.2-7.3 (1H, m), 7.39 (1H, d, J = 3 Hz), 7.69 (1H, d, J = 2 Hz), 8.19 (2H, s), 8.30 (1H, s), 8.43 (1H, d, J = 2 Hz), 8.93 (1H, s).

### [Example 60]

### 5-[4-[5-[7-Fluoro-5-(tetrazol-1-yl)indol-1-ylmethyl]pyrazin-2-yl]piperidin-1-yl]-3-isopropyl-1,2,4-oxadiazole

### (1) 4-[5-[7-Fluoro-5-(tetrazol-1-yl)indol-1-ylmethyl]pyrazin-2-yl]piperidine-1-carbonitrile

To a solution of tert-butyl 4-[5-[7-fluoro-5-(tetrazol-1-yl)indol-1-ylmethyl]pyrazin-2-yl]piperidine-1-carboxylate (Example 34) (84 mg, 0.176 mmol) in dichloromethane (1 mL) was added trifluoroacetic acid (0.9 mL). After stirring at room temperature for 1 hour, the solvent was removed under reduced pressure. The resulting residue was dissolved in dichloromethane (1.8 mL), and wss added sodium hydrogen carbonate (30 mg, 0.351 mmol). To this was added a 3.0 M solution of cyanogen bromide in dichloromethane (0.07 mL, 0.211 mmol) under ice-cooling and stirred at 0°C for 30 minutes. After stirring at room temperature for an additional 16 hours, the reaction mixture was added saturated aqueous sodium hydrogen carbonate solution, and was extracted with chloroform. The organic layer was dried over anhydrous sodium sulfate, and the solvent was removed under reduced pressure. The residue was purified by silica gel column chromatography (chloroform/methanol = 98/2 → 6/1), to give the title compound as a colorless oil (72 mg, yield 100%).
¹H NMR (CDCl₃, 400 MHz): δ= 1.9-2.1 (4H, m), 2.8-2.9 (1H, m), 3.1-3.3 (2H, m), 3.5-3.6 (2H, m), 5.65 (2H, s), 6.7-6.8 (1H, m), 7.27 (1H, dd, J = 2 Hz,12 Hz), 7.39 (1H, d, J = 3 Hz), 7.70 (1H, d, J = 2 Hz), 8.32 (1H, s), 8.41 (1H, s), 8.97 (1H, s).

### (2) 5-[4-[5-[7-Fluoro-5-(tetrazol-1-yl)indol-1-ylmethyl]pyrazin-2-yl]piperidin-1-yl]-3-isopropyl-1,2,4-oxadiazole

Under N₂ atomosphere, 4-[5-[7-fluoro-5-(tetrazol-1-yl)indol-1-ylmethyl]pyrazin-2-yl]piperidine-1-carbonitrile (72 mg, 0.179 mmol) was dissoleved in dioxane (1.5 mL), added dioxane solution of N-hydroxyisobutylimidamide (27 mg, 0.268 mmol) and zinc chloride (37 mg, 0.268 mmol), and stirred at 90°C for 3 hours. After cooling to room temperature, the reaction mixture was added 10% sodium hydroxide solution, and was extracted with ethyl acetate. The organic layer was washed with water and brine, dried over anhydrous sodium sulfate, and the solvent was removed under reduced pressure. The residue was purified by silica gel column chromatography (chloroform/methanol = 20/1, hexane/ethyl acetate 7/1 → 0/100), to give the title compound as a white amorphous (19 mg, yield 22%).
FAB-MS(m/z): 489 (M+1)
¹H NMR (CDCl₃, 400 MHz): δ= 1.28 (6H, d, J = 7 Hz), 1.8-2.0 (4H, m), 2.8-3.1 (2H, m), 3.1-3.3 (2H, m), 4.2-4.3 (2H, m), 5.64 (2H, s), 6.72 (1H, t, J = 3 Hz), 7.2-7.3 (1H, m), 7.39 (1H, d, J = 3 Hz), 7.69 (1H, d, J = 2 Hz), 8.31 (1H, s), 8.43 (1H, d, J = 2 Hz), 8.94 (1H, s).

### [Example 61]

### Pharmacological experiment 1

### (1)Construction of the stable cell line expressing human G-protein coupled receptor 119 (hGPR119)

Human GPR119 gene (NM 178471) was purchased from ATCC (ATCC No. 10807349), and is amplyfied according to PCR to form BamHI site at 5' side and Apa I site at 3' side. The primers were tcctggatccatggaatcatctttctcatt (sequence No. 1) and tcctgggcccttagccatcaaactctgagc (sequence No. 2). The PCR conditions are described below.

The double-stranded DNA was thermally denatured using a DNA polymerase (KOD-Plus-Ver. 2; TOYOBO #KOD-211) at 98°C for 10 seconds in one cycle. The denatured single-stranded DNA was annealed with the primers at 55°C for 30 seconds. The DNA was subjected to an extension reaction at 68°C for 1 minute and 15 seconds. The above-mentioned steps were repeated in 35 cycles. The PCR product was inserted into pcDNA5/FRT/TO (Invitrogen #V6520-20) plasmid. Flp-in T-Rex-293 cells (Invitorogen #R78007) were transfected with the obtained plasmid. The method of transfection was conducted in accordance with the protocol of the product.

### (2) Measurement of intracellular cAMP

The stable cell line expressing human GPR119 prepared in the above-mentioned method was plated on a 96-well plate at the concentration of 2,500 cells/well using Dulbecco's Modified Eagle Medium (DMEM) containing 10% fetal bovine serum (FBS). Twenty-four hours after plating, tetracyclin (Invitrogen #Q10019) was added at the final concentration of 20 ng/mL to induce hGPR119 gene expression. Twenty-four hours after, the medium was removed, and the cells were stimulated with an assay buffer (0.5 mM IBMX PBS (-)) containing the test compound at 37°C for 30 minutes. The amount of the intracellular cAMP was measured using a commercially available kit (HitHunter™ cAMP XS+ Assay: GE Healthcare #90007503) and a reader (FLUOstar Optima: BMG LABTECH). The test compound was dissolved in 100% DMSO, and added at the final concentration of 1%.

### (3) Experimental result

Examination results are shown in Table 12.

**TABLE 12**

| Test compound | EC₅₀ (nM) |
|---|---|
| Example 1 | 313.0 |
| Example 2 | 76.5 |
| Example 3 | 74.6 |
| Example 4 | 62.4 |
| Example 9 | 13.8 |
| Example 10 | 99.8 |
| Example 11 | 67.3 |
| Example 12 | 56.4 |
| Example 13 | 130.2 |
| Example 15 | 126.0 |
| Example 17 | 123.6 |
| Example 18 | 109.5 |
| Example 19 | 82.8 |
| Example 20 | 21.9 |
| Example 21 | 21.4 |
| Example 24 | 111.3 |

As is clear from Table 12, the compounds of example mention showed an excellent GPR119 agonist effect.

### [Example 62]

### Pharmacological experiment 2

The examination was performed by the method similar to Example 61 Pharmacological experiment 1-(1), 1-(2). Those results are shown in Table 13.

**TABLE 13**

| Test compound | EC₅₀ (nM) |
|---|---|
| Example 28 | 7.6 |
| Example 29 | 35.7 |
| Example 30 | 137.2 |
| Example 34 | 2.9 |
| Example 38 | 16.5 |
| Example 42 | 3.1 |
| Example 46 | 95.0 |
| Example 49 | 30.1 |
| Example 50 | 17.4 |
| Example 51 | 5.4 |
| Example 54 | 14.2 |

### [Example 63]

### Pharmacological experiment 3

### Oral glucose tolerance test in normal mice

### (Experimental procedure)

In this experiment, we examined the inhibitory effect of test compound on glycemic excursions after glucose administration in normal mice. The test methods are shown as follows.

Male 9-week-old ICR mice, habituated to the experimental environment for two weeks, were fasted for 18 hours and used to this experiment. Mice were orally administered the test compound or vehicle (polyethylene glycol 400 : ethanol : Tween80=8 : 1 : 1), and after 30 minutes, they were orally given glucose at the dose of 3 g/kg.

Blood was collected at just before the test compound or vehicle administration (-30 min), immediately before glucose challenge (0 min), 20 min, 40 min, 60 min and 120 min after glucose ingestion and then blood glucose levels were determined.

Inhibition rate (%) of the test compound versus vehicle in areas under the glycemic excursion curve between 0 and 120 min after glucose challenge was determined.

### (Experimental result)

**TABLE 14**

| Test compound (1 mg/kg) | Inhibition rate (%) |
|---|---|
| Example 9 | 31.1 |

As is clear from Table 14, the compounds of example mention showed an excellent inhibitory effect of glycemic excursions.

## Claims

1. A compound having the following formula (I) or a pharmaceutically acceptable salt thereof: 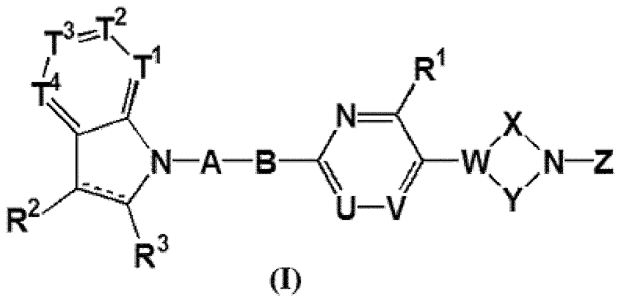
wherein one of T¹, T², T³, and T⁴ is N, and each of the other three independently is CR⁴ or, in the alternative, each of T¹, T², T³, and T⁴ independently is CR⁴, wherein R⁴ is hydrogen, a halogen atom, nitro, cyano, hydroxyl, a C₁₋₈ alkyl group, a C₁₋₈ alkoxy group, a C₁₋₈ alkyl group having one to three halogen atoms, a C₁₋₈ alkoxy group having one to three halogen atoms, phenoxy, an alkoxycarbonyl group containing a C₁₋₈ alkoxy group, carboxyl, carbamoyl, an acyl group containing a C₁₋₈ alkyl group, an alkylaminocarbonyl group containing a C₁₋₈ alkyl group, a dialkylaminocarbonyl group containing C₂₋₁₂ alkyl groups, an alkoxycarbonylmethylcarbonyl group containing a C₁₋₈ alkoxy group, an alkylsulfonylmethyl group containing a C₁₋₈ alkyl group, amino, a C₁₋₈ alkylamino group, a C₂₋₁₂ dialkylamino group, a C₁₋₈ alkylsulfonylamino group, an acylamino group containing a C₁₋₈ alkyl group, a C₁₋₈ alkylsulfinyl group, a C₁₋₈ alkylsulfonyl group, a cycloalkylsulfonyl group containing a three-membered to six-membered ring, sulfamoyl, a C₁₋₈ alkylaminosulfonyl group, a C₂₋₁₂ dialkylaminosulfonyl group, phenylsulfonyl, or a five-membered or six-membered heteroaryl group;
each of R² and R³ independently is hydrogen or a C₁₋₈ alkyl group;
the double line consisting of a solid line and a broken line means a single or double bond;
A is (CH₂)ₘ, C(O), or a bond, wherein m is an integer of 1 to 3;
B is (C(R⁵)H)ₙ, S, O, NR⁶, or a bond, wherein n is an integer of 1 to 3, and each of R⁵ and R⁶ is hydrogen, a C₁₋₈ alkyl group, or a three-membered to six-membered cycloalkyl group, provided that B is neither S, O, nor NR⁶ when A is a bond;
one of U and V is N, and the other is CR⁷, wherein R⁷ is hydrogen, a halogen atom, hydroxyl, a C₁₋₈ alkyl group, a C₁₋₈ alkoxy group, a C₁₋₈ alkyl group having one to three halogen atoms, or a C₁₋₈ alkoxy group having one to three halogen atoms;
R¹ is hydrogen, a halogen atom, hydroxyl, a C₁₋₈ alkyl group, a C₁₋₈ alkoxy group, a C₁₋₈ alkyl group having one to three halogen atoms, or a C₁₋₈ alkoxy group having one to three halogen atoms;
W is C or CR⁸, wherein R⁸ is hydrogen, a halogen atom, hydroxyl, a C₁₋₈ alkyl group, a C₁₋₈ alkoxy group, a C₁₋₈ alkyl group having one to three halogen atoms, or a C₁₋₈ alkoxy group having one to three halogen atoms;
X is a C₁₋₃ alkylene group, which optionally has a substituent or substituents selected from the group consisting of a halogen atom, hydroxyl, a C₁₋₈ alkyl group, a C₁₋₈ alkoxy group, a C₁₋₈ alkyl group having one to three halogen atoms, and a C₁₋₈ alkoxy group having one to three halogen atoms, and X may combine to W with a double bond when W is C;
Y is a C₁₋₃ alkylene group, which optionally has a substituent or substituents selected from the group consisting of a halogen atom, hydroxyl, a C₁₋₈ alkyl group, a C₁₋₈ alkoxy group, a C₁₋₈ alkyl group having one to three halogen atoms, and a C₁₋₈ alkoxy group having one to three halogen atoms;
the substituents of X and Y can be combined to form a C₁₋₃ alkylene group, which optionally has a C₁₋₈ alkyl group; and
Z is C(O)OR⁹, C(O)R¹⁰, SO₂R¹¹, C(O)NR¹²R¹³, CH₂C(O)N(R¹⁴)(R¹⁵), or a five-membered or six-membered heteroaryl group comprising carbon and nitrogen atoms and optionally comprising oxygen or sulfur atom, one of said carbon atoms combining to the nitrogen atom of the neighboring cyclic amine, and said heteroaryl group optionally having a substituent or substituents selected from the group consisting of a halogen atom, a C₁₋₈ alkyl group, a C₁₋₈ alkoxy group, a C₁₋₈ alkyl group having one to three halogen atoms, and a C₁₋₈ alkoxy group having one to three halogen atoms, wherein each of R⁹, R¹⁰, R¹¹, R¹², R¹³, R¹⁴, and R¹⁵ independently is a C₁₋₈ alkyl group, a C₂₋₈ alkenyl group, a three-membered to six-membered cycloalkyl group, phenyl, or a C₁₋₈ alkyl group having phenyl.

2. A compound or a pharmaceutically acceptable salt thereof defined in claim 1, wherein each of T¹, T², T³, and T⁴ independently is CR⁴.

3. A compound or a pharmaceutically acceptable salt thereof defined in claim 1, wherein T¹ is N, and each of T², T³, and T⁴ independently is CR⁴.

4. A compound or a pharmaceutically acceptable salt thereof defined in one of claims 1 to 3, wherein R⁴ is hydrogen, a halogen atom, a C₁₋₈ alkyl group, cyano, an alkoxycarbonyl group containing a C₁₋₈ alkoxy group, a C₁₋₈ alkylsulfonyl group, sulfamoyl, phenylsulfonyl, or a five-membered or six-membered heteroaryl group.

5. A compound or a pharmaceutically acceptable salt thereof defined in one of claims 1 to 3, wherein one of CR⁴ represented by T¹, T², T³, and T⁴ is C-(C₁₋₈ alkylsulfonyl).

6. A compound or a pharmaceutically acceptable salt thereof defined in one of claims 1 to 3, wherein one of CR⁴ represented by T¹, T², T³, and T⁴ is C-(C₁₋₈ alkylsulfonyl), and each of the others of CR⁴ is selected from CH, C-(C₁₋₈ alkyl), or C- (halogeno).

7. A compound or a pharmaceutically acceptable salt thereof defined in one of claims 1 to 3, wherein one of CR⁴ represented by T¹, T², T³, and T⁴ is C-(1-tetrazolyl) or C-(1,2,4-triazol-1-yl).

8. A compound or a pharmaceutically acceptable salt thereof defined in one of claims 1 to 3, wherein one of CR⁴ represented by T¹, T², T³, and T⁴ is C-(1-tetrazolyl) or C-(1,2,4-triazol-1-yl), and each of the others of CR⁴ is selected from CH, C-(C₁₋₈ alkyl), or C-(halogeno).

9. A compound or a pharmaceutically acceptable salt thereof defined in one of claims 1 to 8, wherein each of R² and R³ is hydrogen.

10. A compound or a pharmaceutically acceptable salt thereof defined in one of claims 1 to 9, wherein A is CH₂, and B is a bond.

11. A compound or a pharmaceutically acceptable salt thereof defined in one of claims 1 to 10, wherein U is CH, and V is N.

12. A compound or a pharmaceutically acceptable salt thereof defined in one of claims 1 to 10, wherein U is N, and V is CH.

13. A compound or a pharmaceutically acceptable salt thereof defined in one of claims 1 to 12, wherein each of X and Y is ethylene.

14. A compound or a pharmaceutically acceptable salt thereof defined in one of claims 1 to 12, wherein W is C, and X combines to W with a double bond.

15. A compound or a pharmaceutically acceptable salt thereof defined in one of claims 1 to 14, wherein Z is C(O)OR⁹.

16. A compound or a pharmaceutically acceptable salt thereof defined in claim 15, wherein R⁹ is a C₁₋₈ alkyl group.

17. A compound or a pharmaceutically acceptable salt thereof defined in one of claims 1 to 14, wherein Z is 3-C₁₋₈ alkyl-1,2,4-oxadiazol-5-yl or 5-C₁₋₈ alkyl-1,2,4-oxadiazol-3-yl.

18. A compound or a pharmaceutically acceptable salt thereof defined in one of claims 1 to 14, wherein Z is 5-C₁₋₈ alkylpyrimidin-2-yl.

19. A compound or a pharmaceutically acceptable salt thereof defined in one of claims 1 to 18, wherein each of R¹ and R⁷ is hydrogen.

20. A compound having the following formula (II) or a pharmaceutically acceptable salt thereof: 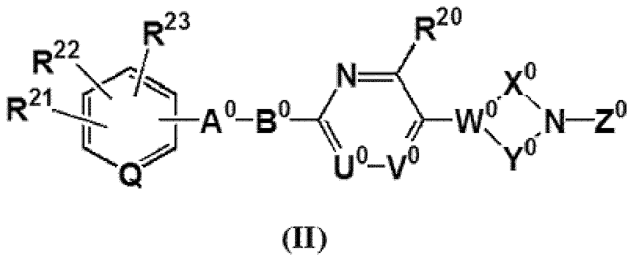
wherein each of R²¹, R²², and R²³ independently is hydrogen, a halogen atom, nitro, cyano, hydroxyl, a C₁₋₈ alkyl group, a C₁₋₈ alkoxy group, a C₁₋₈ alkyl group having one to three halogen atoms, a C₁₋₈ alkoxy group having one to three halogen atoms, phenoxy, an alkoxycarbonyl group containing a C₁₋₈ alkoxy group, carboxyl, carbamoyl, an acyl group containing a C₁₋₈ alkyl group, an alkylaminocarbonyl group containing a C₁₋₈ alkyl group, a dialkylaminocarbonyl group containing C₂₋₁₂ alkyl groups, an alkoxycarbonylmethylcarbonyl group containing a C₁₋₈ alkoxy group, an alkylsulfonylmethyl group containing a C₁₋₈ alkyl group, amino, a C₁₋₈ alkylamino group, a C₂₋₁₂ dialkylamino group, a C₁₋₈ alkylsulfonylamino group, an acylamino group containing a C₁₋₈ alkyl group, a C₁₋₈ alkylsulfinyl group, a C₁₋₈ alkylsulfonyl group, a cycloalkyl-sulfonyl group containing a three-membered to six-membered ring, sulfamoyl, a C₁₋₈ alkylaminosulfonyl group, a C₂₋₁₂ dialkylaminosulfonyl group, phenylsulfonyl, or a five-membered or six-membered heteroaryl group;
Q is N or CH;
A⁰ is (CH₂)ₚ, C(O), S, O, NR²⁴, or a bond, wherein p is an integer of 1 to 3, and R²⁴ is hydrogen, a C₁₋₈ alkyl group, or a three-membered to six-membered cycloalkyl group;
B⁰ is (C(R²⁵)H)_{q}, O, NR²⁶, or a bond, wherein q is an integer of 1 to 3, and each of R²⁵ and R²⁶ is hydrogen, a C₁₋₈ alkyl group, or a three-membered to six-membered cycloalkyl group, provided that B⁰ is neither O nor NR²⁶ when A⁰ is S, O, or NR²⁴, and that B⁰ is not a bond when A⁰ is a bond;
one of U⁰ and V⁰ is N and the other is CR²⁷, wherein R²⁷ is hydrogen, a halogen atom, hydroxyl, a C₁₋₈ alkyl group, a C₁₋₈ alkoxy group, a C₁₋₈ alkyl group having one to three halogen atoms, or a C₁₋₈ alkoxy group having one to three halogen atoms;
R²⁰ is hydrogen, a halogen atom, hydroxyl, a C₁₋₈ alkyl group, a C₁₋₈ alkoxy group, a C₁₋₈ alkyl group having one to three halogen atoms, or a C₁₋₈ alkoxy group having one to three halogen atoms;
W⁰ is C or CR²⁸, wherein R²⁸ is hydrogen, a halogen atom, hydroxyl, a C₁₋₈ alkyl group, a C₁₋₈ alkoxy group, a C₁₋₈ alkyl group having one to three halogen atoms, or a C₁₋₈ alkoxy group having one to three halogen atoms;
X⁰ is a C₁₋₃ alkylene group, which optionally has a substituent or substituents selected from the group consisting of a halogen atom, hydroxyl, a C₁₋₈ alkyl group, a C₁₋₈ alkoxy group, a C₁₋₈ alkyl group having one to three halogen atoms, and a C₁₋₈ alkoxy group having one to three halogen atoms, and X⁰ may combine to W⁰ with a double bond when W⁰ is C;
Y⁰ is a C₁₋₃ alkylene group, which optionally has a substituent or substituents selected from the group consisting of a halogen atom, hydroxyl, a C₁₋₈ alkyl group, a C₁₋₈ alkoxy group, a C₁₋₈ alkyl group having one to three halogen atoms, and a C₁₋₈ alkoxy group having one to three halogen atoms;
the substituents of X⁰ and Y⁰ can be combined to form a C₁₋₃ alkylene group, which optionally has a C₁₋₈ alkyl group; and
Z⁰ is C(O)OR²⁹, C(O)R³⁰, SO₂R³¹, C(O)NR³²R³³, CH₂C(O)N(R³⁴)(R³⁵), or a five-membered or six-membered heteroaryl group comprising carbon and nitrogen atoms and optionally comprising oxygen or sulfur atom, one of said carbon atoms combining to the nitrogen atom of the neighboring cyclic amine, and said heteroaryl group optionally having a substituent or substituents selected from the group consisting of a halogen atom, a C₁₋₈ alkyl group, a C₁₋₈ alkoxy group, a C₁₋₈ alkyl group having one to three halogen atoms, and a C₁₋₈ alkoxy group having one to three halogen atoms, wherein each of R²⁹, R³⁰, R³¹, R³², R³³, R³⁴, and R³⁵ independently is a C₁₋₈ alkyl group, a C₂₋₈ alkenyl group, a three-membered to six-membered cycloalkyl group, phenyl, or a C₁₋₈ alkyl group having phenyl.

21. A compound or a pharmaceutically acceptable salt thereof defined in claim 20, wherein Q is CH.

22. A compound or a pharmaceutically acceptable salt thereof defined in claim 20 or 21, wherein each of R²¹, R²², and R²³ independently is hydrogen, a halogen atom, a C₁₋₈ alkyl group, cyano, an alkoxycarbonyl group containing a C₁₋₈ alkoxy group, a C₁₋₈ alkylsulfonyl group, sulfamoyl, phenylsulfonyl, or a five-membered or six-membered heteroaryl group.

23. A compound or a pharmaceutically acceptable salt thereof defined in claim 20 or 21, wherein one of R²¹, R²², and R²³ is a C₁₋₈ alkylsulfonyl group.

24. A compound or a pharmaceutically acceptable salt thereof defined in claim 20 or 21, wherein one of R²¹, R²², and R²³ is a C₁₋₈ alkylsulfonyl group, and each of the others is selected from hydrogen, a C₁₋₈ alkyl group, or a halogen atom.

25. A compound or a pharmaceutically acceptable salt thereof defined in claim 20 or 21, wherein one of R²¹, R²², and R²³ is 1-tetrazolyl or 1,2,4-triazol-1-yl.

26. A compound or a pharmaceutically acceptable salt thereof defined in claim 20 or 21, wherein one of R²¹, R²², and R²³ is 1-tetrazolyl or 1,2,4-triazol-1-yl, and each of the others is selected from hydrogen, a C₁₋₈ alkyl group, or a halogen atom.

27. A compound or a pharmaceutically acceptable salt thereof defined in one of claims 20 to 26, wherein A⁰ is O, and B⁰ is CH₂.

28. A compound or a pharmaceutically acceptable salt thereof defined in one of claims 20 to 27, wherein U⁰ is CH, and V⁰ is N.

29. A compound or a pharmaceutically acceptable salt thereof defined in one of claims 20 to 27, wherein U⁰ is N, and V is CH.

30. A compound or a pharmaceutically acceptable salt thereof defined in one of claims 20 to 29, wherein each of X⁰ and Y⁰ is ethylene.

31. A compound or a pharmaceutically acceptable salt thereof defined in one of claims 20 to 29, wherein W⁰ is C, and X⁰ combines to W⁰ with a double bond.

32. A compound or a pharmaceutically acceptable salt thereof defined in one of claims 20 to 31, wherein Z⁰ is C(O)OR²⁹.

33. A compound or a pharmaceutically acceptable salt thereof defined in claim 32, wherein R²⁹ is a C₁₋₈ alkyl group.

34. A compound or a pharmaceutically acceptable salt thereof defined in one of claims 20 to 31, wherein Z⁰ is 3-C₁₋₈ alkyl-1,2,4-oxadiazol-5-yl or 5-C₁₋₈ alkyl-1,2,4-oxadiazol-3-yl.

35. A compound or a pharmaceutically acceptable salt thereof defined in one of claims 20 to 31, wherein Z⁰ is 5-C₁₋₈ alkylpyrimidin-2-yl.

36. A compound or a pharmaceutically acceptable salt thereof defined in one of claims 20 to 35, wherein each of R²⁰ and R²⁷ is hydrogen.

37. An agent for treating diabetes containing a compound or a pharmaceutically acceptable salt thereof defined in one of claims 1 to 36 as an active ingredient.

38. A GPR119 agonist containing a compound or a pharmaceutically acceptable salt thereof defined in one of claims 1 to 36 as an active ingredient.
